# EUROPEAN PATENT APPLICATION

(11) **EP 4 393 494 A1**
(43) Date of publication of application: **03.07.2024**
(21) Application number: 22216932.8
(22) Date of filing: 28.12.2022
(51) Int. Cl.: A61K 31/70, A61P 31/04, A23K 20/195

(54) **COMPOUND FOR USE IN THE TREATMENT AND/OR PREVENTION OF A CLOSTRIDIUM DIFFICILE INFECTION IN A PIG**

(71) Applicant: HUVEPHARMA, 2600 Antwerpen (BE)
(72) Inventor: PETKOV, Spas, 2600 Antwerpen (BE); HAUTEKIET, Veerle, 2600 Antwerpen (BE)
(74) Representative: Gevers Patents

(57) **Abstract**

The invention relates to compounds and compositions for use in the treatment and/or prevention of a *Clostridium difficile* infection in a pig.

## Description

### FIELD OF INVENTION

This present invention relates to compounds for use in the treatment and/or prevention of a *Clostridium difficile* infection in a pig.

### BACKGROUND OF THE INVENTION

*Clostridioides difficile* (*Clostridium difficile, C. difficile, C. diff.* or *CD*) is a gram-positive, spore-forming anaerobic bacterium which is found widely in soil, water, and in the intestinal tract of various mammals, birds, and reptiles. The species was transferred from the genus *Clostridium* to *Clostridioides* in 2016, thus giving it the binomial *Clostridioides difficile.* This new classification reflects the taxonomic differences between this species and members of the genus *Clostridium,* while maintaining the common name as *Clostridium difficile.*

*C. difficile* can be responsible for various *C. difficile* infections (CDI), also called *C. difficile* associated diseases (CDAD). It is already an established cause of antibiotic-associated diarrhea in mice and rabbits, antibiotic-associated diarrhea and pseudomembranous colitis in humans, chronic diarrhea in dogs, typhlitis in hamsters, typhlitis and nosocomial diarrhea in adult horses or of enterocolitis in foals. Germination of *C. difficile* spores in the large intestine is usually uneventful, but if the normal flora has not been established or has been disrupted in some ways, *C. difficile* will establish, multiply and produce toxins. Infections often occur as a result of exposure to antimicrobials, but dietary changes may also potentiate infection (Songer *et al.,* DOI: 10.1016/j.anaerobe.2005.09.001).

In swine, CDI is one of the most important enteric diseases of the neonate. Diagnostic findings support a role for C. *difficile* in producing important pathologic changes in the intestines of piglets (Jones *et al.,* DOI: 10.1136/vr.112.11.253). *C. difficile* intestinal colonization occurs within the first hours-of-life, and nearly one hundred percent of piglets in some herds are colonized within 48 hours-of-birth; however, clinical disease is only observed in specific circumstances. Transmission is fecal-oral and the bacteria and spores are often endemic within the farm environment. Studies have shown that ambient air and the environment are the main source of C. *difficile* to neonatal pigs (Hopman *et al.,* DOI: 10.1016/j.vetmic.2010.10.013). It has also been shown that approximately 25% of sows tested were actively shedding *C. difficile* during lactation (Norman *et al.,* doi: 10.1128/AEM.05007-11). Despite the majority of *C. difficile* cases occur in the first week-of-life, Arruda and colleagues demonstrated that 10 day-old piglets are as susceptible as neonatal piglets when challenged with a toxigenic strain of C. *difficile* (Arruda *et al.,* DOI: 10.1016/j.anaerobe.2013.04.010).

Cultures of affected tissues commonly yield heavy growth of C. *difficile* and presence of toxins A and B (TcdA and TcdB, respectively), known as "large clostridial toxins". Studies suggest a 93.7% correlation between clinical, pathologic and bacteriologic diagnosis, on the one hand, and toxin detection on the other. Toxin detection in rectal swabs is also an acceptable protocol, in that results are 92.8% correlated with those from examination of intestinal contents. Numerous virulence factors associated with CDI have been described including surface proteins (pili and capsule) and exotoxins, although it is well accepted that exotoxins A and B are the major virulence factors associated with disease development. Some strains of C. *difficile* also produce an ADP-ribosylating binary toxin; however, the role of this toxin on the pathogenesis of disease has not been elucidated (Davies *et al.,* DOI: 10.1042/BJ20110106). Toxin A (307 kDa) is an enterotoxin which causes fluid accumulation in the intestine and toxin B (270 kDa) is a cytotoxin which is highly cytopathic. Both toxins are internalized by target cells and disrupt the cytoskeleton by enzymatic attack on intracellular targets resulting in substantial tissue damage (Borriello *et al.,* DOI: 10.1.suppl_3.13).

Gross lesions frequently includes moderate to severe mesocolonic edema, sometimes accompanied by hydrothorax and/or ascites. Colonic contents may be pasty-to-watery and yellow, although some piglets are constipated or obstipated. Focal suppuration and segmental necrosis are seen on microscopic examination of fecal and colonic lamina propria, and exudation of neutrophils and fibrin into the lumen gives rise to the so-called "volcano" lesions. Gross lesions are not pathognomonic, and diagnosis must be confirmed by culture or demonstration of either toxin A or B and histopathology. The genes of toxins A and B are identified readily by PCR. The toxins can also be detected directly in suspensions of intestinal contents by commercially available enzyme immunoassays.

*C. difficile* may be the most important uncontrolled cause of neonatal diarrhea in pigs (Songer *et al.,* DOI: 10.1016/j.anaerobe.2005.09.001). Mortality rates associated with outbreaks of CDI are variable; however, rates as high as 50% have been reported in suckling piglets. Retardation of growth and lower weaning weights, approximately 0.5 kg lighter on average, have been reported in recovered piglets (Songer, DOI: 10.1079/ahr200492).

The characterization of the microbiota diversity inhabiting particular mucosal surfaces or other body sites has been an active topic of research in recent years, due to the involvement in many vital processes. The gut microbiota of mammals has been shown to confer health benefits to the host through the production of digestible food components, inhibition and prevention of colonization by pathogens, and development and maintenance of the host immune system. The microbiota composition from the pigs' gastrointestinal tract (GIT) has been subject of many investigations. Piglets are first exposed to microbes at birth and they will eventually be colonized with different microbial populations by constant exposure to microbes. Early gut colonization is critically for both morphological and immunological development of the GIT. During growth, the microbiome changes exhibiting increased diversity, a relevant indicator of GIT health. Once established, the microbiota ultimately reaches an equilibrated community composition that is essential in maintaining the health of the animal. However, under several pathological conditions or antimicrobial treatments, disruption of the microbiota may occur leading to a dysbiosis state. Moreover, there are other important factors that may impact the GIT microbiota composition such as diet, age and the environment (e.g. habitat, geographical range) where animals are raised. Intensive farming has led to pigs being mostly raised indoors, where they are exposed to a limited diversity of microorganisms (Correa-Fiz et al., DOI: 10.1038/s41598-019-49897-1).

Translation of results of microbiome studies from a species to another can be very difficult. In the last decades, the advancement of DNA sequencing technology has made it possible to investigate variation of the entire microbial community. For example, the host-associated microbiome varies between species as well as within and between natural populations. Interspecific variation in microbiomes exists both in captivity (Ley *et al.,* doi: 10.1126/science. 1155725) and in the wild (Phillips *et al.,* DOI: 10.1111/j.1365-294X.2012.05568.x). Between-population variation in the microbial composition has been observed in rodents (Kreisinger *et al.,* doi: 10.1098/rstb.2014.0295) and primates (Amato *et al.,* DOI: 10.1038/ismej.2013.16), including humans (Suzuki and Worobey, DOI: 10.1098/rsbl.2013.1037). Within-population variation in the microbiome has also been observed in humans (Arumugam *et al.,* doi: 10.1038/nature09944), chimpanzees (Moeller *et al.,* doi: 10.1038/ncomms2159), and house mice (Wang *et al.,* doi: 10.1073/pnas. 1402342111).

The microbiota of the mammalian gut is a complex ecosystem, the composition of which is greatly influenced by host genetics and environmental factors. Despite recent intensive research on variation in the gut microbiota composition within and between vertebrate species, the factors that generate the gut microbial diversity are still not sufficiently understood. Additionally, it is largely unknown how often closely related species differ in the gut microbiota composition and thus how widespread the effect of the gut microbiota in speciation (Sottas *et al.,* doi: 10.1186/s12862-021-01773-1).

*C. difficile* spores are highly resistant to physical and chemical agents such as farm cleaning procedures and most common disinfectants (Fawley *et al*., doi: 10.1086/519201) and can survive for many months in the environment (Speight *et al.,* doi: 10.1016/j.jhin.2011.05.016).

Based on minimum inhibitory concentration (MIC) determinations of antimicrobial agents for 80 isolates of C. *difficile,* it has been suggested that erythromycin, tetracycline, and tylosin may be useful against certain isolates for the treatment of suckling piglets, while a large proportion of isolates showed in vivo resistance. Also, it appears that tiamulin and virginiamycin may help to reduce only moderately levels of the organism in adult swine (Post *et al.,* doi: 10.101 6/j. an aerobe. 2004.01.003).

A non-toxigenic strain of C. *difficile* (NTCD), named Z31, has been tested in hamster and piglet experimental models as a strategy to prevent CDI. Results suggest that the use of non-toxigenic *C. difficile* as a probiotic can reduce the severity of histologic lesions and the quantity of toxins detected in neonatal piglets (Junior *et al.,* DOI: 10.1016/j.vetmic.2019.02.026).

However, treatments with antimicrobials and use of probiotics has yielded mainly unsatisfactory results. To date, there are no commercially available products to prevent and/or treat efficiently CDI in pigs, especially in piglets. This underlines the difficulty of translating results from in vitro experiments or other animal models towards the actual situation in living pigs and piglets for this particular disease. In spite of the importance of CDI in pigs, in particular in neonatal piglets, there are still major gaps in the knowledge regarding basic concepts of epidemiology, pathogenesis and prevention in swine.

In view of the above there is a need for a compound for use in the treatment and/or prevention of CDI in pigs, in particular in piglets, that may overcome the drawbacks of prior art i.e. which is improved (more efficient), in particular which significantly decreases the incidence and the severity of CDI in pigs.

### SUMMARY OF THE INVENTION

The inventors have now surprisingly found that it is possible to fulfil the above mentioned needs by the provision of the compounds of the invention, in particular bambermycin and analogous compounds. While bambermycin and analogous compounds are currently used in poultry and cattle as feed additives and growth promoters, the inventors have surprisingly found that these compounds are effective in preventing and treating a *C. difficile* infection in pigs.

It is an objective of the present invention to provide compounds to effectively treat and/or prevent a C. *difficile* infection in pigs, particularly in piglets. In particular, the compounds and methods of the invention allow to significantly reduce the incidence and the severity caused by this infection.

The present invention relates to a compound for use in the treatment and/or prevention of a *C. difficile* infection in a pig, preferably in a piglet, wherein said compound is at least one of the compounds according to general formulae (I), (II), (III) or (IV), or the pharmaceutically acceptable salt, pharmaceutically acceptable solvate, isomer or mixture thereof; 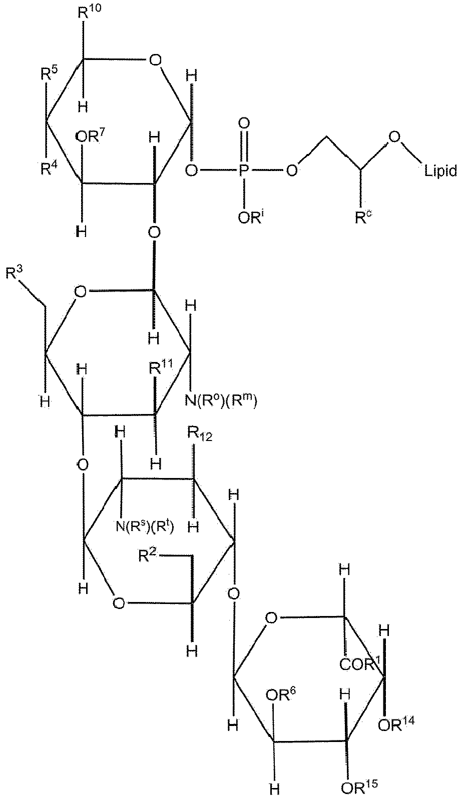 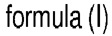 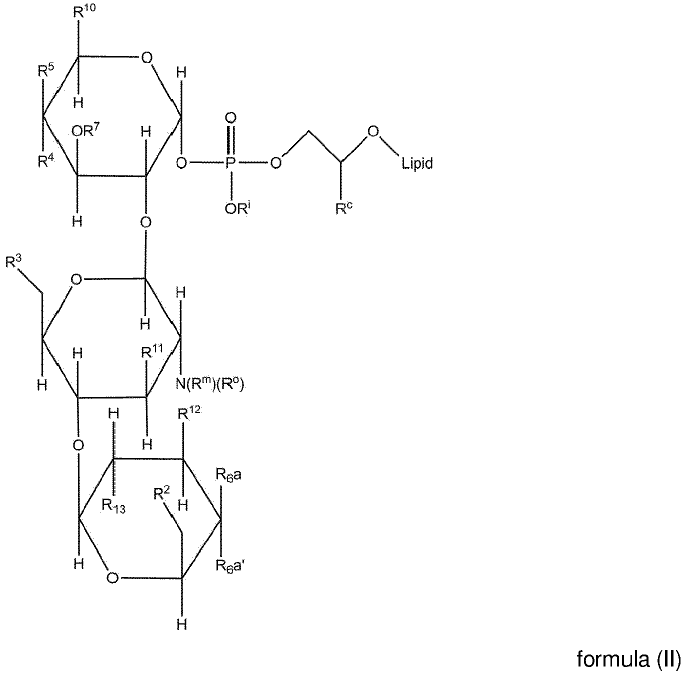 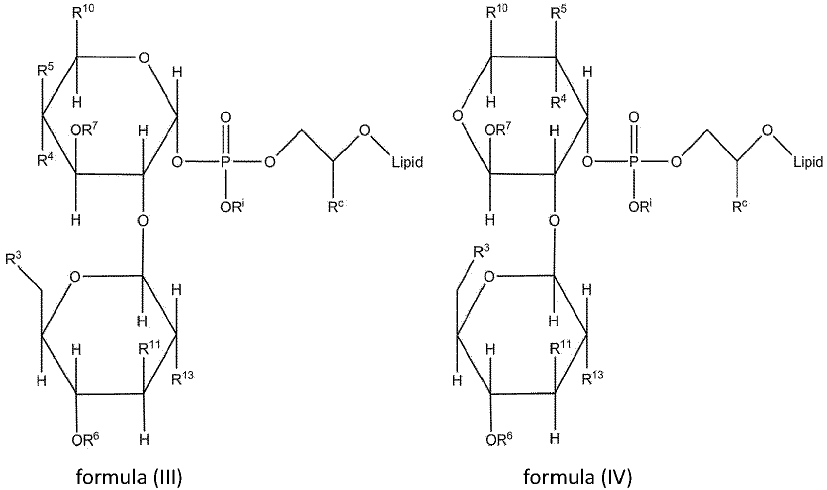 wherein
- each of R¹ is independently selected from -OH or -N(R^{1a})(R^{1b}), wherein each of R^{1a} and R^{1b}, independently from each other and at each occurrence, are selected from the group consisting of H, an amino protecting group, aryl, heteroaryl, aliphatic and a heteroaliphatic group,
- each of R², R⁴, R⁵, R¹¹ and R¹², independently from each other and at each occurrence, are selected from the group consisting of H, -OR^{z}, -N(R^{z})₂, aryl, heteroaryl, aliphatic, and a heteroaliphatic group, wherein R^{z} is independently selected from the group consisting of H, an hydroxyl protecting group, an amino protecting group, aryl, heteroaryl, aliphatic, heteroaliphatic and a carbohydrate moiety,
- each of R³ is independently selected from the group consisting of H, -OH, -NH₂, -SH, OR^{w}, - NH(R^{w}), -N(R^{w})₂, -SR^{w}, -O(C=O)R^{w}, -NH(C=O)R^{w}, -O(C=NH)R^{w}, -NH(C=NH)R^{w}, -S(C=NH))R^{w}, -NH(C=S)R^{w}, -S(C=O)R^{w}, -O(C=S)R^{w}, -S(=S)R^{w}, aryl, heteroaryl, aliphatic and a heteroaliphatic group, wherein R^{w} is selected from the group consisting of a carbohydrate moiety, aryl, heteroaryl, aliphatic, and heteroaliphatic group,
- each of R⁶, R¹⁴ and R¹⁵, independently from each other and at each occurrence are selected from the group consisting of H, hydroxyl protecting group, aryl, heteroaryl, aliphatic, heteroaliphatic group and carbohydrate moiety,
- each of R⁷ is independently selected from the group consisting of H, C(=O)N(R^{z'})₂, -C(=O)OR^{z'} , an hydroxyl protecting group, aryl, heteroaryl, aliphatic, heteroaliphatic group and carbohydrate moiety, wherein R^{z'} is independently selected from the group consisting of H, an hydroxyl protecting group, an amino protecting group, aryl, heteroaryl, aliphatic, and a heteroaliphatic group,
- each of R¹⁰ is independently selected from the group consisting of -C(=O)N(R^{l})(R^{p}), -C(=O)OR^{k}, and -CH₂OR^{k}, wherein R^{l} and R^{p} are independently from each other and at each occurrence selected from the group consisting of H, an amino protecting group, aryl, heteroaryl, aliphatic, heteroaliphatic group and a carbohydrate moiety, wherein R^{k} is independently selected from the group consisting of H, an hydroxyl protecting group, aryl, heteroaryl, aliphatic, heteroaliphatic group and a carbohydrate moiety,
- each of R¹³ is independently selected from the group consisting of -OH and -N(R^{o"})(R^{m"}) , wherein R^{o"} and R^{m"} independently from each other and at each occurrence, are selected from the group consisting of H, -C(=O)R^{h}, an amino protecting group, aryl, heteroaryl, aliphatic and heteroaliphatic group, wherein R^{h} is selected from the group consisting of a carbohydrate moiety, aryl, heteroaryl, aliphatic and heteroaliphatic group,
- each of (R°) and (R^{m}), independently from each other and at each occurrence, are selected from the group consisting of H, -C(=O)R^{w'}, an amino protecting group, aryl, heteroaryl, aliphatic and heteroaliphatic group, wherein R^{w'} is selected from the group consisting of a carbohydrate moiety, aryl, heteroaryl, aliphatic and heteroaliphatic group;
- each of (R^{s}) and (R^{t}), independently from each other and at each occurrence are selected from the group consisting of H, -C(=O)R^{w"}, an amino protecting group, aryl, heteroaryl, aliphatic and heteroaliphatic group, wherein R^{w"} is selected from the group consisting of a carbohydrate moiety, aryl, heteroaryl, aliphatic and heteroaliphatic group;
- each of R^{6a} and R^{6a'}, independently from each other and at each occurrence are selected from H or -OH, preferably R^{6a} is H and R^{6a'} is -OH;
- each of R^{c} is independently selected from the group consisting of H, halogen, heteroaryl, -OR^{q}, -N(R^{q})₂, -SR^{q}, NO₂, -NC, -CN, -N₃, -N(R^{q})=NR^{q}, -CHO, -C(=O)R^{q}, -C(=S)R^{q}, C(=NR^{q})R^{q}, - C(=O)OR^{q}, -C(=NR^{q})OR^{q}, -C(=NR^{q})N(R^{q})₂, -C(=O)N(R^{q})₂, -C(=S)OR^{q}, -C(=O)SR^{q}, -C(=S)SR^{q}, - P(=O)(OR^{q})₂, -S(=O)(OR^{q}), -S(=0)₂(OR^{q}), -P(=O)N(R^{q})₂, -P(=O)₂N(R^{q})₂, -C(=0)NR'S(=O)₂R^{q}, - S(=O)N(R^{q})₂ and -S(=O)₂N(R^{q})₂, wherein each of R^{q} is independently selected from the group consisting of H, aliphatic, heteroaliphatic, aryl, heteroaryl, and an hydroxyl protecting group,
- each of Rⁱ is independently selected from the group consisting of H, an hydroxyl protecting group, aliphatic, heteroaliphatic, aryl, and heteroaryl,
- each Lipid is independently selected from H or a C₁₋₃₀ aliphatic moiety, wherein 0 to 10 methylene units are optionally replaced with -O-, -NR^{x}-, -S-, -C(=O)-, -C(=NR^{x})-, -S(=O)-, - S(=O)₂-, -N=N-, -C=N-, -C(R^{y})=C(R^{y'})-, -N-O-, an arylene, or an heteroarylene moiety, wherein each of R^{x} is independently selected from the group consisting of H, aliphatic, heteroaliphatic, aryl, heteroaryl, or an amino protecting group, and wherein each of R^{y} and R^{y'}, independently from each other and at each occurrence are selected from the group consisting of H, aliphatic, heteroaliphatic, aryl and heteroaryl group.

The present invention further relates to a compound for use in the treatment and/or prevention of a C. *difficile* infection in a pig, preferably in a piglet, comprising administering a daily dose of 10 to 1000 mg, preferably 50 to 500 mg of the compound as defined in any one of the embodiments herein.

The present invention further relates to a compound for use in the treatment and/or prevention of a C. *difficile* infection in a pig, preferably in a piglet, comprising administering a daily dose of 2.5 to 350 mg/kg bodyweight, preferably of 10 to 150 mg/kg bodyweight of the compound.

The present invention further relates to a compound, as defined in any one of the embodiments presented herein, for use in the treatment and/or prevention of a C. *difficile* infection in a pig, preferably in a piglet, by oral administration of the compound, preferably by drenching.

The present invention further relates to a compound, as defined in any one of the embodiments presented herein, for use in the treatment and/or prevention of a C. *difficile* infection in a pig, preferably in a piglet, wherein the compound is administered at least 2 consecutive days.

The present invention further relates to a composition for use in the treatment and/or prevention of a *C. difficile* infection in a pig, preferably in a piglet, the composition comprising the compound as defined in any one of the embodiments presented herein.

The present invention further relates to a composition for use in the treatment and/or prevention of a C. *difficile* infection in a pig, preferably in a piglet, wherein the treatment and/or the prevention comprises orally administering the compound as defined in one of the embodiments presented herein in a daily dose of 10 to 150 mg/kg bodyweight for at least 2 consecutive days.

The present invention further relates to a composition for use in the treatment and/or prevention of a *C. difficile* infection in a pig, preferably in a piglet, the composition comprising bambermycin.

The present invention further relates to a pig feed composition comprising a compound as defined in one of the embodiments presented herein for use in the treatment and/or the prevention of a *Clostidium difficile* infection in a pig.

### DETAILED DESCRIPTION OF THE INVENTION

It is further understood that all definitions and preferences as described for the compounds of the invention above equally apply for this embodiment and all further embodiments, as described below.

As used in the foregoing and hereinafter, the following definitions apply unless otherwise noted.

A "moenomycin" as used herein refers to a member of the moenomycin family of antibiotics. Moenomycins are phosphoglycolipid antibiotics, originally obtained from *Streptomyces.* The most notable moenomycins, and those preferred in the present invention, include moenomycin A, moenomycin A₁₂, moenomycin C₁, moenomycin C₃, moenomycin C₄, pholipomycin, AC326-alpha, nosokomycin A, nosokomycin B, nosokomycin C, and nosokomycin D.

"Bambermycin" is a known complex of compounds, primarily composed of moenomycins A and C. It can be obtained from *Streptomyces bambergiensis* and *Streptomyces ghanaensis Streptomyces ederensis, Streptomyces geysiriensis* and related strains or biosynthesized. Bambermycin is also known as flavophospholipol and is available under its commercial name Flavomycin or Gainpro. Bambermycin, flavophospholipol and Flavomycin are used interchangeably herein.

"Ppm" and percentages as used herein refer to mass fractions, unless its context clearly dictates otherwise.

The term "aliphatic" includes both saturated and unsaturated, nonaromatic, straight chain (i.e., unbranched), branched, acyclic, and cyclic hydrocarbon chain having 1 to 30 carbon atoms, which are optionally substituted with one or more groups including but not limited to alkyl, alkynyl, alkenyl, aryl, halide, nitro, amino, ester, ketone, aldehyde, hydroxy, carboxylic acid, or alkoxy. According to certain embodiments, as used herein, C_{F-G} aliphatic defines a C_{F-G} aliphatic group having F to G carbon atoms, e.g. C₁₋₁₂ aliphatic defines an aliphatic group containing 12 carbon atoms, C₁₋₁₀ aliphatic defines an aliphatic group containing 1 to 10 carbon atoms.

As will be appreciated by one of ordinary skilled in the art, "aliphatic" is intended herein to include, but is not limited to alkyl, alkenyl, and alkynyl moieties. As used herein the term "alkyl" "alkenyl", and "alkynyl" have the broadest meaning generally understood in the art, and may include a moiety which is linear, branched, cyclic (cycloalkyl, cycloalkenyl, cycloalkynyl) or a combination thereof.

The term "alkyl", alone or in combination means an alkane-derived radical, which may be a straight chain alkyl, branched alkyl or cyclic alkyl, containing from 1 to 30 carbon atoms, unless otherwise specified. The straight chain or branched alkyl group is attached at any available point to produce a stable compound. Alkyl also includes a straight chain or branched alkyl group that contains or is interrupted by a cycloalkyl portion. According to certain embodiments C_{A-B} alkyl defines a straight or branched alkyl radical having from A to B carbon atoms, e.g. C₁₋₁₅ alkyl defines a straight or branched alkyl radical having from 1 to 15 carbon atoms, C₁₋₁₂ alkyl defines a straight or branched alkyl radical having from 1 to 12 carbon atoms, C₁₋₆ alkyl defines a straight or branched alkyl radical having from 1 to 6 carbon atoms such as for example methyl, ethyl, 1-propyl, 2-propyl, I-butyl, 2-butyl, 2-methyl-1-propyl. According to certain embodiments a cyclic C_{C-D} alkyl defines a cyclic alkyl radical having from C to D carbon atoms, e.g. C₃₋₆ cyclic alkyl.

The term "alkenyl", alone or in combination, means a straight or branched hydrocarbon containing 1-30 carbon atoms, unless otherwise specified and at least one carbon to carbon double bond. Examples of alkenyl groups include ethenyl, propenyl, isopropenyl, butenyl, cyclohexenyl, cyclohexenylalkyl and the like. Alkenyl also includes a straight chain or branched alkenyl group that contains or is interrupted by a cycloalkyl portion. Carbon to carbon double bonds may be either contained within a cycloalkyl portion or within a straight chain or branched portion. According to certain embodiments C_{H-I} alkenyl defines a straight or branched alkenyl radical having from H to I carbon atoms, e.g. C₁₋₆ alkenyl defines a straight or branched alkenyl radical having from 1 to 6 carbon atoms.

The term "alkynyl" alone or in combination means a straight, branched or cyclic hydrocarbon containing 1 to 30 carbon atoms containing at least one carbon to carbon triple bond. Examples of alkynyl groups include ethynyl, propynyl, butynyl and the like.

As used herein, the term "heteroaliphatic," refers to an aliphatic moiety, as defined herein, which includes both saturated and unsaturated, nonaromatic, straight chain (i.e.,unbranched), branched, acyclic, cyclic (i.e., heterocyclic), or polycyclic hydrocarbons having 1 to 30 carbon atoms, unless specified otherwise, which are optionally substituted with one or more groups, including but not limited to alkyl, alkynyl, alkenyl, aryl, halide, nitro, amino, ester, ketone, aldehyde, hydroxy, carboxylic acid, or alkoxy and that contain one or more hetero atoms such as oxygen, sulfur, nitrogen, phosphorus, or silicon atoms, e.g., in place of carbon atoms. In certain embodiments, heteroaliphatic moieties are substituted by independent replacement of one or more of the hydrogen atoms thereon with one or more substituents. As will be appreciated by one of ordinary skilled in the art, "heteroaliphatic" is intended herein to include heteroalkyl, heterocycloalkyl, heterocycloalkenyl, and heterocycloalkynyl moieties. Thus, as used herein, the term "heteroalkyl" includes straight, branched and cyclic alkyl groups that contain one or more one or more hetero atoms such as oxygen, sulfur, nitrogen, phosphorus, or silicon atoms, e.g., in place of carbon atoms. An analogous convention applies to other generic terms such as "heteroalkenyl", "heteroalkynyl", and the like. Furthermore, as used herein, the terms "heteroalkyl", "heteroalkenyl", "heteroalkynyl", and the like encompass both substituted and unsubstituted groups.

As used herein, the term "aryl", alone or in combination means any carbon-based aromatic group including, but not limited to, phenyl, tolyl, xylyl, cumenyl, naphthyl, anthracenyl etc., optionally carbocyclic fused with a cycloalkyl or heterocyclyl of preferably 5-7, more preferably 5-6, ring members and/or optionally substituted with 1 to 5 groups or substituent. An aryl may be optionally substituted with one or more groups including but not limited to alkyl, alkynyl, alkenyl, aryl, halide, nitro, amino, ester, ketone, aldehyde, hydroxy, carboxylic acid, or alkoxy whereby the substituent is attached at one point to the aryl or whereby the the substituent is attached at two points to the aryl to form a bicyclic system e.g. benzodioxole, benzodioxan, benzimidazole.

The term "heteroaryl", alone or in combination means a monocyclic aromatic ring structure containing 5 or 6 ring atoms, or a bicyclic aromatic group having 8 to 10 atoms, containing one or more, preferably 1-4, more preferably 1-3, heteroatoms independently selected from the group O, S, and N, and optionally substituted with 1 to 5 groups or substituents including but not limited alkyl, alkynyl, alkenyl, aryl, halide, nitro, amino, ester, ketone, aldehyde, hydroxy, carboxylic acid, or alkoxy Heteroaryl is also intended to include oxidized S or N, such as sulfinyl, sulfonyl and N-oxide of a tertiary ring nitrogen. A carbon or nitrogen atom is the point of attachment of the heteroaryl ring structure such that a stable aromatic ring is retained. More specifically the term heteroaryl includes, but is not limited to, pyridyl, furanyl, thiophenyl, thiazolyl, isothiazolyl, triazolyl, imidazolyl, isoxazolyl, pyrrolyl, pyrazolyl, pyrimidinyl, benzofuranyl, isobenzofuranyl, benzothiazolyl, benzoisothiazolyl, benzotriazolyl, indolyl, isoindolyl, benzoxazolyl, quinolyl, isoquinolyl, benzimidazolyl, benzisoxazolyl, benzothiophenyl, dibenzofuran, and benzodiazepin-2-one-5-yl, and the like.

According to a certain embodiment of the present invention, the Lipid is an unsaturated C₄₋₂₀ hydrocarbon chain, wherein 1 to 10 methylene units are replaced by -C(R^{y})=C(R^{y'})- wherein each of R^{y} and R^{y'}, independently from each other and at each occurrence, are selected from the group consisting of H, aliphatic, heteroaliphatic, aryl, or heteroaryl; and wherein said hydrocarbon chain is optionally substituted with a C₁₋₆ alkyl, a C₃₋₆ cyclic alkyl, C₁₋₆ alkenyl or hydroxyl group, preferably the Lipid is a C₄₋₂₀ hydrocarbon chain, wherein 1 to 5 methylene units are replaced by -C(R^{y})=C(R^{y'})-wherein each of R^{y} and R^{y'}, independently from each other and at each occurrence, are selected from the group consisting of H, C₁₋₆ alkyl and C₁₋₆ alkenyl and wherein said hydrocarbon chain is optionally substituted with a C₁₋₆ alkyl, a C₃₋₆ cyclic alkyl, C₁₋₆ alkenyl or hydroxyl group, more preferably the Lipid is chosen among following formulae: 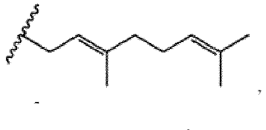 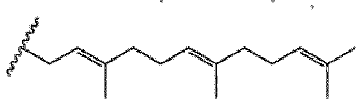 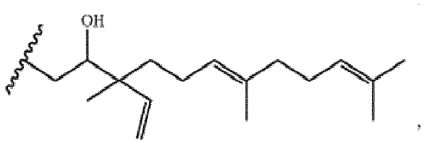 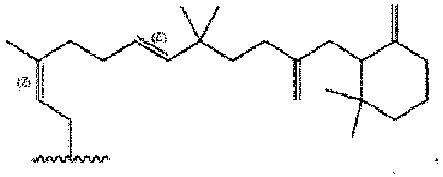 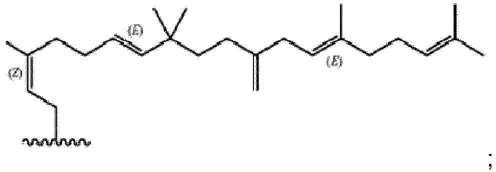 and

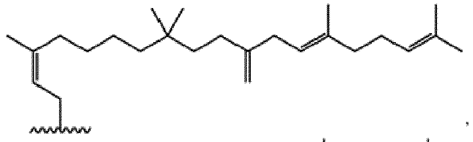

Even more preferred, the Lipid is chosen among following formulae:

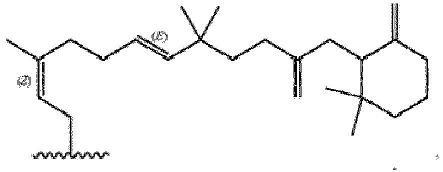

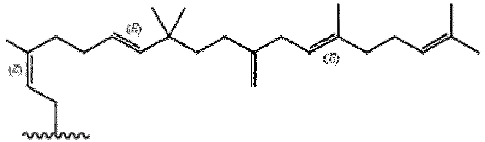

According to a certain embodiment of the present invention, the Lipid is a saturated hydrocarbon chain according to general formula Z: 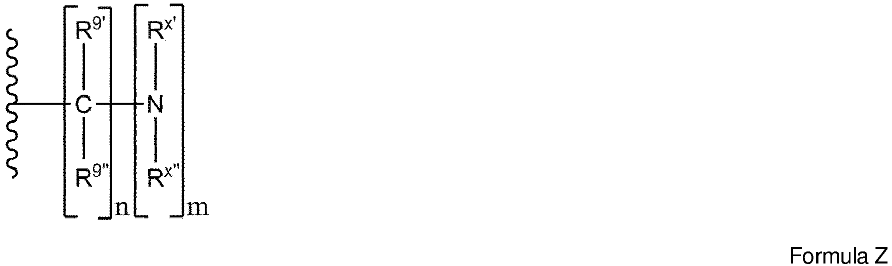 wherein
- each of R^{9'} and R^{9"} are independently from each other and at each occurrence selected from the group consisting of H, -OH and C₁₋₆ alkyl,
- each of R^{x'} and R^{x"} , independently from each other and at each occurrence, are selected from H, a C₁₋₁₅ alkyl and an aryl wherein said aryl is optionally further substituted by a C₁₋₁₅ alkyl, and wherein n = 4 to 30, and m = 0 or 1

Preferably, the saturated hydrocarbon chain is chosen among following formulae: 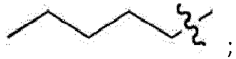 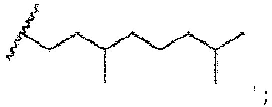 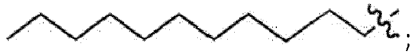 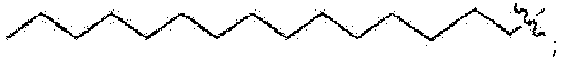 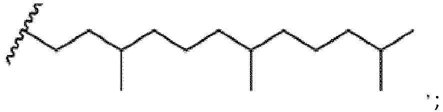 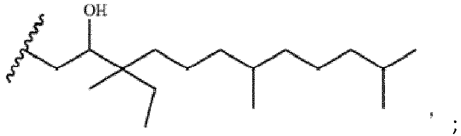 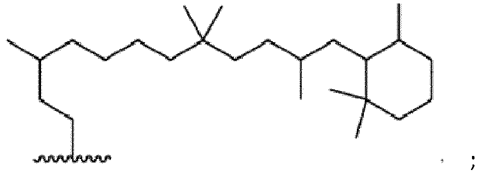 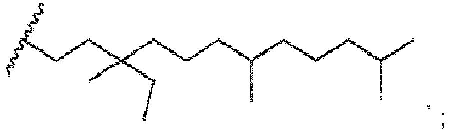 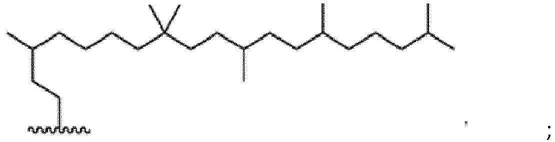 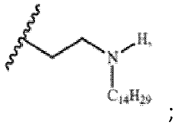 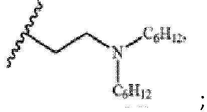 and

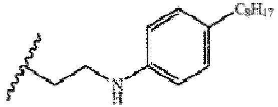

According to a certain embodiment of the present invention, R¹ is independently selected from -OH or -N(R^{1a})(R^{1b}), wherein each of R^{1a} and R^{1b}, independently from each other and at each occurrence, are selected from the group consisting of H, C₁₋₂₀ aliphatic and C₁₋₂₀ heteroaliphatic group, preferably R¹ is OH, NH₂, or NH(R^{1b}), wherein R1b is a C₁₋₁₀ cycloalkyl group or C₁₋₁₀ cycloalkenyl which is optionally substituted with an halide, hydroxyl or alkoxy, more preferably R¹ is -NH(R^{1b}), wherein R^{1b} is of following formula:

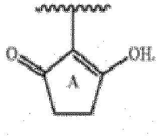

According to a certain embodiment of the present invention, R³ is independently selected from the group consisting of H, -OH and -OR^{w}, wherein R^{w} is selected from the group consisting of a carbohydrate moiety, wherein said carbohydrate moiety is independently selected from the group consisting of D- and L- monosaccharides, such as notably D-erythrose, L-erythrose, D-threose, L-threose, L- erythrulose, D- erythrulose, D-arabinose, L-arabinose, D- deoxyribose, L- deoxyribose, D-lyxose, L-lyxose, D-ribose, L-ribose, D-ribulose, L-ribulose, D-xylose, L-xylose, D-xylulose, L-xylulose, D-allose, L- allose, D-altrose, L-altrose, D-galactose, L-galactose, D-glucose, L-glucose, D-gulose, L-gulose, D-idose, L-idose, D-mannose, L-mannose, D-talose, L-talose, D-fructose, L-fructose, D-psicose, L-psicose, D-sorbose, L-sorbose, D-tagatose, L-tagatose, D-fucose, L-fucose, D-rhamnose and L-rhamnose, disaccharides, such as notably sucrose, lactose, trehalose, and maltose, trisaccharides such as notably acarbose, raffinose, and melezitose, more preferably, R³ is -OR^{w}, wherein R^{w} is a D-monosaccharide such as notably D-erythrose, D-threose, D- erythrulose, D-arabinose, D-deoxyribose, D-lyxose, D-ribose, D-ribulose, D-xylose, D-xylulose, D-allose, D-altrose, D-galactose, D-glucose, D-gulose, D-idose, D-mannose, D-talose, D-fructose, D-psicose, D-sorbose, D-tagatose, D-fucose, and D-rhamnose, even more preferably, R³ is -OR^{w}, wherein R^{w} is independently selected from the group consisting of D-glucose, D-galactose, D-allose, D-altrose, D-mannose, D-iodose, D-galose or D-talose, most preferably, R³ is -OR^{w}, wherein R^{w} is D-glucose.

According to a certain embodiment of the present invention, each of R², R⁴, R⁵, R¹¹ and R¹², independently from each other and at each occurrence, are selected from the group consisting of H, OR^{z}, a C₁₋₁₂ aliphatic, C₁₋₁₂ heteroaliphatic and a carbohydrate moiety, wherein R^{z} is selected from the group consisting of H, hydroxyl protecting group, C₁₋₁₂ aliphatic, C₁₋₁₂ heteroaliphatic, aryl and heteroaryl, and wherein said carbohydrate moiety is selected from the group consisting of D-monosaccharides such as notably D-erythrose, D-threose, D- erythrulose, D-arabinose, D-deoxyribose, D-lyxose, D-ribose, D-ribulose, D-xylose, D-xylulose, D-allose, D-altrose, D-galactose, D-glucose, D-gulose, D-idose, D-mannose, D-talose, D-fructose, D-psicose, D-sorbose, D-tagatose, D-fucose, and D-rhamnose, preferably each R², R⁴, R⁵, R¹¹ and R¹², independently from each other and at each occurrence, are selected from the group consisting of H, OR^{z}, a hydroxyl protecting group, C₁₋₆ alkyl and a carbohydrate moiety, wherein R^{z} is selected from the group consisting of H, a hydroxyl protecting group and C₁₋₆ alkyl, and wherein said carbohydrate moiety is selected from the group consisting of D-glucose, D-galactose, D-allose, D-altrose, D-mannose, D-iodose, D-galose and D-talose, more preferably R², R⁴, R¹¹ and R¹², independently from each other and at each occurrence, are selected from H or OR^{z}, wherein R^{z} is selected from H or C₁₋₆ alkyl, more preferably R⁵ is a C₁₋₆ alkyl, even more preferably R² is H, R⁴, R¹¹ and R¹² are -OH and R⁵ is -CH₃;

According to a certain embodiment of the present invention, each of R⁶, R¹⁴ and R¹⁵, independently from each other and at each occurrence, are selected from the group consisting of H, hydroxyl protecting group, C₁₋₁₂ aliphatic and C₁₋₁₂ heteroaliphatic group, preferably R⁶, R¹⁴ and R¹⁵, independently from each other and at each occurrence, are selected from the group consisting of H or an hydroxyl protecting group, more preferably R⁶, R¹⁴ and R¹⁵, independently from each other and at each occurrence, are H.

According to a certain embodiment of the present invention, R⁷ is -C(=O)N(R^{z'})₂ group, wherein each of R^{z'} is independently selected from the group consisting of R^{z'} is selected from the group consisting of H, aryl, heteroaryl and C₁₋₁₂ alkyl, preferably each of R^{z'} is independently selected from H or C₁₋₁₂ alkyl, more preferably each of R^{z'} is independently selected from the group consisting of H, methyl, ethyl and propyl, most preferably each of R^{z'} is independently H.

According to a certain embodiment of the present invention, each of R¹⁰ is independently selected from-C(=O)N(R^{l})(R^{p}) or -C(=O)OR^{k}, wherein R^{l} and R^{p} are independently from each other and at each occurrence, selected from the group consisting of H, an amino protecting group, aryl, heteroaryl, C₁₋₁₂ aliphatic and C₁₋₁₂ heteroaliphatic group , and wherein R^{k} is independently selected from the group consisting of H, an hydroxyl protecting group, aryl, heteroaryl, C₁₋₃₀ aliphatic and C₁₋₃₀ heteroaliphatic group, preferably each of R¹⁰ is independently selected from -C(=O)N(R^{l})(R^{p}) or - C(=O)OR^{k}, wherein R^{l} and R^{p} are independently from each other and at each occurrence, selected from the group consisting of H, an amino protecting group, aryl, heteroaryl and C₁₋₆ alkyl, wherein each of R^{k} is independently selected from the group consisting of H, an hydroxyl protecting group and C₁₋₆ alkyl, more preferably, each of R¹⁰ is independently selected from -C(=O)N(R^{l})(R^{p}) or - C(=O)OR^{k}, wherein R^{l} and R^{p}, independently from each other and at each occurrence, are selected from the group consisting of H, an amino protecting group, methyl, ethyl, and propyl, wherein each of R^{k} is independently selected from the group consisting of H, an hydroxyl protecting group, methyl, ethyl, and propyl, most preferably, R¹⁰ is -C(=O)NH₂ or -C(=O)OH.

According to a certain embodiment of the present invention, each of R¹³ is independently selected from the group consisting of -OH and -N(R^{o"})(R^{m"}), wherein R^{o"} and R^{m"}, independently from each other and at each occurrence, are selected from the group consisting of H, -C(=O)R^{h}, an amino protecting group, C₁₋₁₂ aliphatic and C₁₋₁₂ heteroaliphatic group, wherein R^{h} is independently selected from a C₁₋₁₂ aliphatic or C₁₋₁₂ heteroaliphatic group, preferably, R^{o"} and R^{m'}, independently from each other and at each occurrence, are selected from the group consisting of H, -C(=O)R^{h} and C₁₋₁₂ alkyl, wherein R^{h} is C₁₋₁₂ alkyl, more preferably R^{o"} and R^{m"}, independently from each other and at each occurrence, are selected from the group consisting of H, -C(=O)R^{h} and C₁₋₆ alkyl, wherein R^{h} is C₁₋₆ alkyl, most preferably R^{o"} is H and R^{m'} is -C(=O)CH₃.

According to a certain embodiment of the present invention, each of (R°) and (R^{m}), independently from each other and at each occurrence, are selected from the group consisting of H, -C(=O)R^{w'}, an amino protecting group, C₁₋₁₂ aliphatic and C₁₋₁₂ heteroaliphatic group, wherein R^{w'} is independently selected from the group consisting of a C₁₋₁₂ aliphatic and C₁₋₁₂ heteroaliphatic group, preferably (R°) and (R^{m}), independently from each other and at each occurrence, are selected from the group consisting of H, -C(=O)R^{w'}, an amino protecting group and C₁₋₁₂ alkyl, wherein R^{w'} is a C₁₋₁₂ alkyl, more preferably (R°) and (R^{m}), independently from each other and at each occurrence, are selected from the group consisting of H, -C(=O)R^{w'}, and C₁₋₆ alkyl, wherein R^{w'} is a C₁₋₆ alkyl, most preferably (R°) is H and (R^{m}) is -C(=O)CH₃.

According to a certain embodiment of the present invention, each of (R^{s}) and (R^{t}), independently from each other and at each occurrence are selected from the group consisting of H, -C(=O)R^{w"}, an amino protecting group, C₁₋₁₂ aliphatic and C₁₋₁₂ heteroaliphatic group, wherein R^{w"} is independently selected from a C₁₋₁₂ aliphatic or C₁₋₁₂ heteroaliphatic group, preferably (R^{s}) and (R^{t}), independently from each other and at each occurrence, are selected from the group consisting of H, -C(=O)R^{w"}, an amino protecting group and C₁₋₁₂ alkyl, wherein R^{w"} is C₁₋₁₂ alkyl, more preferably (R^{s}) and (R^{t}), independently from each other and at each occurrence, are selected from the group consisting of H, -C(=O)R^{w"}, and C₁₋₆ alkyl, wherein R^{w"} is C₁₋₆ alkyl, most preferably (R^{s}) is H and (R^{t}) is -C(=O)CH₃.

According to a certain embodiment of the present invention, each of R^{c} is independently selected from the group consisting of H, -C(=O)OR^{q}, -C(=O)SR^{q}, -C(=S)OR^{q}, -C(=O)SR^{q}, -C(=S)SR^{q}, wherein each of R^{q} is independently selected from the group consisting of H, C₁₋₁₂ alkyl, aryl, heteroaryl and an hydroxyl protecting group, preferably each of R^{c} is independently selected from the group consisting of H, -C(=O)OR^{q}, -C(=O)SR^{q}, and -C(=O)SR^{q}, wherein each of R^{q} is independently selected from the group consisting of H, C₁₋₆ alkyl and an hydroxyl protecting group, more preferably each of R^{c} is independently selected from H or -C(=O)OR^{q}, wherein each of R^{q} is independently selected from the group consisting of H, methyl, ethyl and propyl, most preferably R^{c} is -C(=O)OH.

According to a certain embodiment of the present invention, each of Rⁱ is independently selected from the group consisting of H, an hydroxyl protecting group, C₁₋₁₂ aliphatic, C₁₋₁₂ heteroaliphatic, aryl and heteroaryl, preferably Rⁱ is independently selected from the group consisting of H, an hydroxyl protecting group, C₁₋₁₂ alkyl, aryl and heteroaryl, more preferably Rⁱ is independently selected from the group consisting of H, an hydroxyl protecting group and C₁₋₆ alkyl, even more preferably Rⁱ is independently selected from the group consisting of H, an hydroxyl protecting group, methyl, ethyl, and propyl, most preferably Rⁱ is H.

The compounds for use of the present invention, as detailed above, have several centers of chirality and exist as stereochemically isomeric forms. The term "stereochemically isomeric forms" as used herein defines all the possible compounds made up of the same atoms bonded by the same sequence of bonds but having different three-dimensional structures which are not interchangeable, which the anti-inflammatory compound as specified herein, may possess.

Unless otherwise mentioned or indicated, the chemical designation of the compounds for use, as detailed above, encompasses the mixture of all possible stereochemically isomeric forms, which said compounds for use may possess. Said mixture may contain all diastereomers and/or enantiomers of the basic molecular structure of said compounds for use. All stereochemically isomeric forms of the compounds of the present invention both in pure form or mixed with each other are intended to be embraced within the scope of the present invention.

In a preferred embodiment, the compound of the invention is a moenomycin. Therefore, the present invention provides a moenomycin for use in the prevention and/or treatment of a C. *difficile* infection in a pig, preferably in a piglet. Preferred compounds for use in the invention are compounds according to formula (Ia) [compounds for use of class I, herein after] or the pharmaceutically acceptable salt, pharmaceutically acceptable solvate, isomer or mixture thereof, 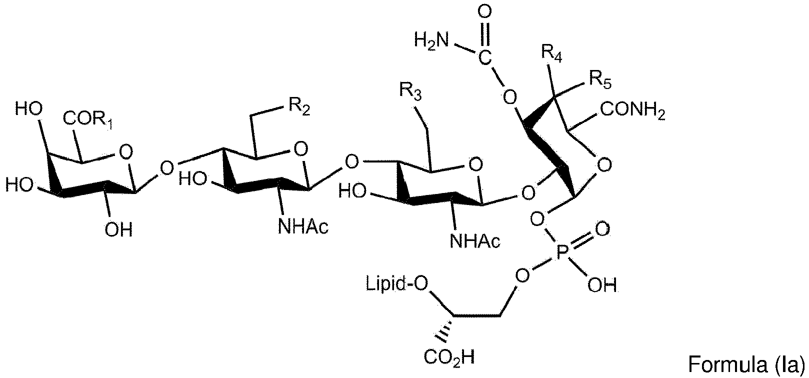 wherein
- each of R¹ is independently selected from OH, NH₂, or NH(R^{1b}), wherein R1b is a C1-10 cycloalkyl group or C1-10 cycloalkenyl which is optionally substituted with an halide, hydroxyl or alkoxy, more preferably R1 is -NH(R^{1b}), wherein R^{1b} is of following formula: 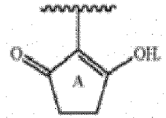
- each of R³ is independently selected from the group consisting of H, -OH and -OR^{w}, wherein R^{w} is D-glucose;
- each of R² and R⁴, independently from each other and at each occurrence are selected from H or -OH;
- each of R⁵ is independently selected from the group consisting of H, -OH and -CH₃;
- the Lipid is chosen among following Formula L₁ or Formula L₂:

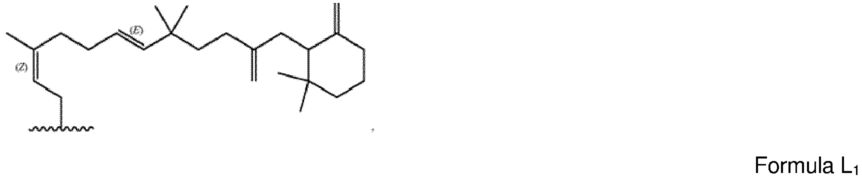

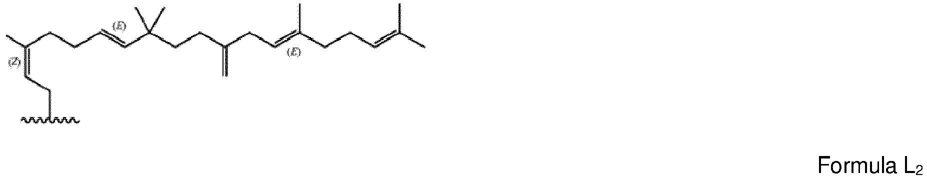

More preferably, the present invention provides a composition for use, wherein the composition comprises a mixture of compounds of class I, as detailed above.

Non limitative examples of compositions for use suitable for the invention include bambermycin, commercially available as Flavomycin^{®}.

A preferred compound for use of class I is moenomycin A according to formula (Ib), or the pharmaceutically acceptable salt, pharmaceutically acceptable solvate, isomer or mixture thereof:

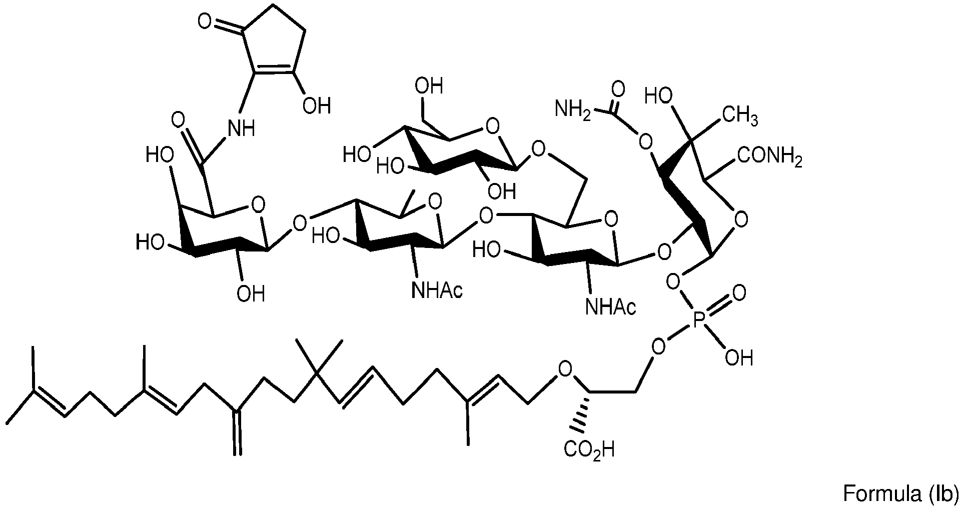

According to an alternative embodiment of the present invention, compounds for use are compounds according to formula (IIa) [compounds for use of class II, herein after] or the pharmaceutically acceptable salt, pharmaceutically acceptable solvate, isomer or mixture thereof: 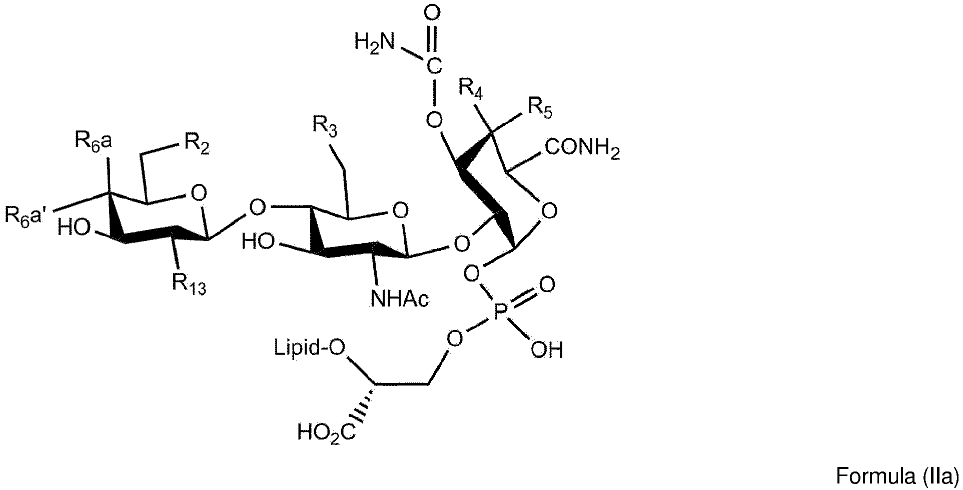 wherein
- R3 is H or -OH;
- each of R² and R⁴, independently from each other and at each occurrence, are selected from H or -OH;
- each of R^{6a} and R^{6a'}, independently from each other and at each occurrence, are selected from H or -OH, preferably R^{6a} is H and R^{6a'} is -OH;
- R⁵ is H, -OH or CH₃;
- R¹³ is -OH or -NHC(=O)CH₃
- the Lipid is chosen among following Formula L₃, Formula L₄ or Formula L₅:

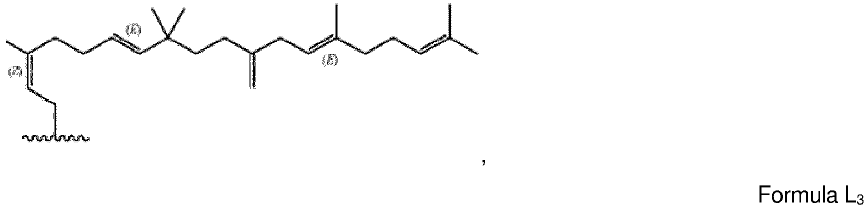

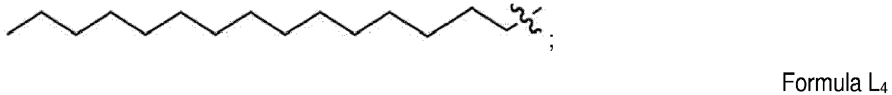

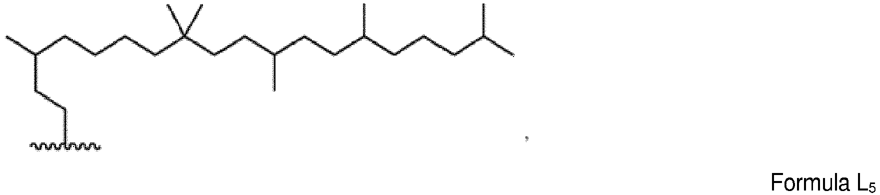

According to an alternative embodiment of the present invention, compounds for use according to the invention are compounds according to formula (Illa) [compounds for use of class III, herein after] or the pharmaceutically acceptable salt, pharmaceutically acceptable solvate, isomer or mixture thereof: 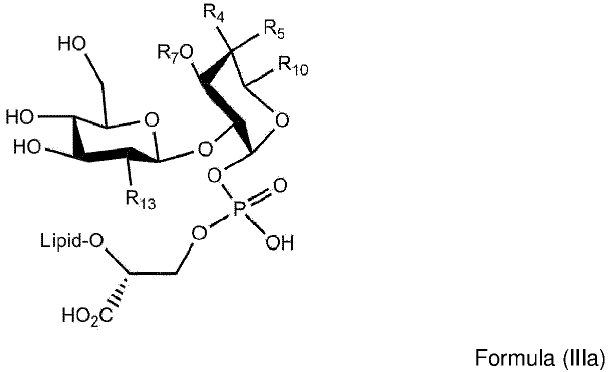 wherein
- each of R⁴ and R⁵, independently from each other and at each occurrence are selected from the group consisting of H, -CH₃ and -OH;
- R₇ is independently selected from H or -C(=O)NH₂, preferably, R₇ is C(=O)NH₂;
- R¹⁰ is independently selected from -C(=O)NH₂ or -C(=O)OH, preferably, R¹⁰ is C(=O)NH_{2.};
- R¹³ is independently selected from the group consisting of -OH or -NHC(=O)CH₃, preferably R¹³ is --NHC(=O)CH₃
- the Lipid is chosen among following Formula L₆, Formula L₇ or Formula L₈:

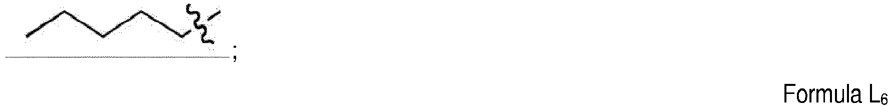

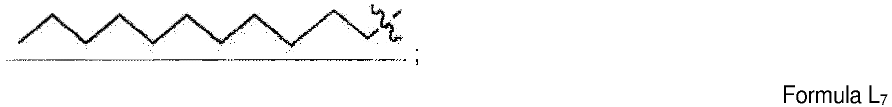

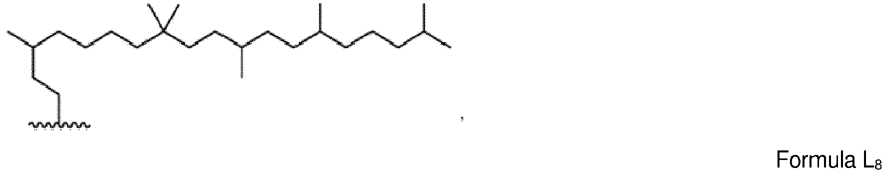

For therapeutic use, salts of the compounds for use of the present invention, as detailed above, are those wherein the counter-ion is pharmaceutically acceptable, which salts can be referred to as pharmaceutically acceptable acid and base addition salts. However, salts of acids and bases that are non-pharmaceutically acceptable may also find use, for example, in the preparation or purification of a pharmaceutically acceptable compound. All salts, whether pharmaceutically acceptable or not, are included within the ambit of the present invention.

The pharmaceutically acceptable acid and base addition salts as mentioned hereinabove are meant to comprise the therapeutically active non-toxic acid and base addition salt forms that the compounds for use of the present invention, as detailed above, are able to form. The pharmaceutically acceptable acid addition salts can conveniently be obtained by treating the base form with such appropriate acid in an anion form. Appropriate anions comprise, for example, trifluoroacetate, acetate, benzenesulfonate, benzoate, bicarbonate, bitartrate, bromide, calcium edetate, camsyiate, carbonate, chloride, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, methylsulfate, mucate, napsylate, nitrate, pamoate (embonate), pantothenate, phosphate/diphosphate, polygalacturonate, salicylate, stearate, subacetate, succinate, sulfate, tannate, tartrate, teoclate, triethiodide, and the like. The counterion of choice can be introduced using ion exchange resins. Conversely said salt forms can be converted by treatment with an appropriate base into the free base form.

The compounds for use as specified herein, containing an acidic proton may also be converted into their nontoxic metal or amine addition salt forms by treatment with appropriate organic and inorganic bases in a cation form. Appropriate basic salts comprise those formed with organic cations such as benzathine, chloroprocaine, choline, diethanolamine, ethylenediamine, meglumine, procaine, and the like; and those formed with metallic cations such as aluminum, calcium, lithium, magnesium, potassium, sodium, zinc, and the like. Conversely said salt forms can be converted by treatment with an appropriate acid into the free form.

The term addition salt as used hereinabove also comprises the solvates which the compounds for use, as specified herein, as well as the salts thereof, are able to form. Such solvates are for example hydrates, alcoholates and the like.

### COMPOSITIONS

The present invention further relates to a composition for use in the treatment and/or prevention of a C. *difficile* infection in a pig, preferably in a piglet, the composition comprising a compound as defined above and as defined in any one of the embodiments presented herein, in particular the compound for use of any of the classes (I) to (III), as defined above.

In the rest of the text, the expression "compound " or "compound according to the invention" is understood, for the purposes of the present invention, both in the plural and the singular, that is to say that the inventive composition may comprise one or more than one "compound according to the invention".

In a particular embodiment, the present invention provides moenomycin A, as well as compositions comprising moenomycin A, for use in the prevention and/or treatment of a C. *difficile* infection in a pig, preferably in a piglet. As a further embodiment of the present invention, the compound according to the invention is the compound with the International Union of Pure and Applied Chemistry (IUPAC) name (2S,3S,4R,5R,6R)-5-[(2S,3R,4R,5S,6R)-3-acetamido-5-[(2S,3R,4R,5S,6R)-3-acetamido-4-hydroxy-6-methyl-5-[(2R,3R,4S,5R,6S)-3,4,5-trihydroxy-6-[(2-hydroxy-5-oxocyclopenten-1-yl)carbamoyl]oxan-2-yl]oxyoxan-2-yl]oxy-4-hydroxy-6-[[(2R,3R,4S,5S,6R)-3,4,5-trihydroxy-6-(hydroxymethyl)oxan-2-yl]oxymethyl]oxan-2-yl]oxy-4-carbamoyloxy-6-[[(2R)-2-carboxy-2-[(2E,6E,13E)-3,8,8,14,18-pentamethyl-11-methylidenenonadeca-2,6,13,17-tetraenoxy]ethoxy]-hydroxyphosphoryl]oxy-3-hydroxy-3-methyloxane-2-carboxylic acid with CAS number 11015-37-5.

The present invention further relates to a composition for use, the composition comprising bambermycin. Bambermycin can be obtained from *Streptomyces bambergiensis Streptomyces ghanaensis Streptomyces ederensis, Streptomyces geysiriensis* and related strains. Bambermycin is also known as flavophospholipol. The extraction of bambermycin is e.g. disclosed in GB1 139589 and GB1068639, both incorporated by reference. In said references, bambermycin is referred to as "the antibiotic Moenomycin". Bambermycin is a complex of compounds, primarily composed of moenomycins A and C. Moenomycins are classified as phosphoglycolipids based on their structural composition. They contain 3-phosphoglyceric acid, a unique structural element among bacterial secondary metabolites.

As an embodiment of the present invention, the composition comprising the compound of the present invention is an antibiotic moenomycin-containing complex obtained from cultures of *Streptomyces bambergiensis, Streptomyces ghanaensis, Streptomyces ederensis, Streptomyces geysiriensis* and related strains. In a particular embodiment, the composition of the invention comprises at least 5 active components, namely moenomycins A, A₁₂, C₁, C₃ and C₄; preferably moenomycin A being the major component. In another embodiment, the composition of the invention comprises at least 800 mg/g moenomycins. In a further embodiment, said composition comprises:
- at least 50% moenomycin A, preferably at least 65%;
- less than 15% moenomycin A₁₂, preferably less than 10%;
- less than 15% moenomycin C₁, preferably less than 10%;
- less than 20% moenomycin C₃, prerably less than 15%;
- less than 20% moenomycin C₄, preferably less than 15%.

### COMPOUNDS AND/OR COMPOSITIONS FOR USE IN THE TREATMENT AND/OR PREVENTION OF A C. DIFFICILE INFECTION IN A PIG

The present invention further relates to a compound as defined in any one of the embodiments presented herein, for use in the treatment and/or prevention of a C. *difficile* infection in a pig. In a particular embodiment, the pig is a female pig, more in particular a sow. In a further embodiment, the present invention relates to a compound as defined in any one of the embodiments presented herein, for use in the treatment and/or prevention of a C. *difficile* infection in a sow. Therefore, in a particular embodiment, the compound of the invention is administered to a sow, more in particular it is administered to a sow within 0 to 4 weeks after farrowing.

More preferably, the present invention further relates to a compound as defined in any one of the embodiments presented herein, for use in the treatment and/or prevention of a C. difficile infection in a piglet, more preferably in a piglet of 20 days old or less, still more preferably in a piglet of 15 days old or less, still more preferably in a piglet of 7 days-old or less, still more preferably in a newborn piglet. In a particular embodiment, the compound or composition of the invention is administered at least once within 48 hours after the piglet is born, more in particular within 24 hours.

In a preferred embodiment, the treatment and/or prevention comprises administering a daily dose of about 10 to about 1000 mg of the compound. In a further embodiment, the treatment and/or prevention comprises administering a daily dose of about 50 to about 500 mg of the compound.

In a particular embodiment, the treatment and/or prevention comprises administering a daily dose of about 10 to 100 mg of the compound, preferably of about 25 to 75 mg, more preferably of about 40 to 60 mg, still more preferably of about 45 to 55 mg, still more preferably of about 50 mg.

In another particular embodiment, the treatment and/or prevention comprises administering a daily dose of about 100 to 1000 mg of the compound, preferably of about 250 to 750 mg, more preferably of about 400 to 600 mg, still more preferably of about 450 to 550 mg, still more preferably of about 500 mg.

In a particular embodiment, the treatment and/or prevention comprises administering a daily dose of about 10 to about 1000 mg of the compound, preferably of about 50 to 500 mg of the compound to a piglet, more preferably to a piglet of 20 days old or less, still more preferably to a piglet of 15 days old or less, still more preferably to a piglet of 7 days old or less, still more preferably to a newborn piglet.

In a preferred embodiment, the compound is administered to a piglet which weights between 2 and 10 kg, more preferably between 2 and 8 kg, still more preferably between 2 and 6 kg, still more preferably between 2 and 5 kg, still more preferably between 3 and 4 kg.

In a further embodiment, the treatment and/or prevention comprises administering a daily dose of about 2.5 to about 350 mg of the compound according to the invention per kg bodyweight, in particular about 10 to about 150 mg/kg bodyweight.

In another particular embodiment of the present invention, the treatment and/or prevention comprises administering a daily dose of about 25 to about 350 mg/kg bodyweight, more in particular about 50 to about 250 mg/kg bodyweight, still more in particular about 100 to about 200 mg/kg bodyweight, in particular about 125 to about 175 mg/kg bodyweight, more in particular about 150 mg/kg bodyweight.

In another particular embodiment, the treatment and/or prevention comprises administering a daily dose of about 2.5 to about 35 mg/kg bodyweight, more in particular about 5 to about 25 mg/kg bodyweight, still more in particular about 10 to about 20 mg/kg bodyweight, in particular about 12.5 to about 17.5 mg/kg bodyweight, more in particular about 15 mg/kg bodyweight.

In another embodiment, the treatment and/or prevention comprises administering the compound or composition according to the invention for at least 2 consecutive days.

In a preferred embodiment of the present invention, the treatment and/or prevention comprises a first administration of said daily dose of the compound and a second administration of said daily dose of the compound, said second administration being performed between 18 and 30 hours, preferably between 20 and 28 hours, more preferably between 22 and 26 hours, still more preferably about 24 hours post said first administration.

In a further embodiment of the present invention, said first administration of said daily dose of the compound is given to a piglet, more preferably to a piglet of 20 days old or less, still more preferably to a piglet of 15 days old or less, still more preferably to a piglet of 7 days old or less, still more preferably to a newborn piglet.

According to certain embodiments of the present invention, said daily dose of the compound of said first administration is of about 100 to 1000 mg of the compound, preferably of about 250 to 750 mg, more preferably of about 400 to 600 mg, still more preferably of about 450 to 550 mg, still more preferably of about 500 mg, and said daily dose of the compound of said second administration is of about 100 to 1000 mg of the compound, preferably of about 250 to 750 mg, more preferably of about 400 to 600 mg, still more preferably of about 450 to 550 mg, still more preferably of about 500 mg.

According to other embodiments of the present invention, said daily dose of the compound of said first administration is of about 100 to 1000 mg of the compound, preferably of about 250 to 750 mg, more preferably of about 400 to 600 mg, still more preferably of about 450 to 550 mg, still more preferably of about 500 mg, and said daily dose of the compound of said second administration is of about 10 to 100 mg of the compound, preferably of about 25 to 75 mg, more preferably of about 40 to 60 mg, still more preferably of about 45 to 55 mg, still more preferably of about 50 mg.

According to other particular embodiments of the present invention, said daily dose of the compound of said first administration is of about 10 to 100 mg of the compound, preferably of about 25 to 75 mg, more preferably of about 40 to 60 mg, still more preferably of about 45 to 55 mg, still more preferably of about 50 mg, and said daily dose of the compound of said second administration is of about 10 to 100 mg of the compound, preferably of about 25 to 75 mg, more preferably of about 40 to 60 mg, still more preferably of about 45 to 55 mg, still more preferably of about 50 mg.

Still according to other embodiments, said daily dose of the compound of said first administration is of about 10 to 100 mg of the compound, preferably of about 25 to 75 mg, more preferably of about 40 to 60 mg, still more preferably of about 45 to 55 mg, still more preferably of about 50 mg, and said daily dose of the compound of said second administration is is of about 100 to 1000 mg of the compound, preferably of about 250 to 750 mg, more preferably of about 400 to 600 mg, still more preferably of about 450 to 550 mg, still more preferably of about 500 mg.

For the avoidance of doubt, if an embodiment of the invention refers to a specific amount of a compound of the invention, it refers to a specific amount of any of the compounds and preferred compounds mentioned herein, e.g. moenomycins or moenomycin A, or to a specific amount of a mixture of compounds and preferred compounds mentioned herein, e.g. a specific amount of bambermycin.

The invention further relates to a compound for use in the treatment and/or prevention of a *C. difficile* infection in a pig, preferably in a piglet, in particular the compounds for use of any of the classes (I) to (III), as defined above, wherein the compound is administered orally.

Preferably, the compound is orally administered by drenching. More preferably, the compound is orally administered by getting an animal to swallow the compound, the compound being preferably comprised in a solution (drench).

More preferably, the administration of the compound by drenching comprises:
- pour the compound, preferably comprised in a solution, into a bottle, said bottle comprising a neck and optionally a tube arranged to be connected to the neck;
- hold the pig, preferably the piglet, in a sitting position with its head slightly up;
- insert the neck or tube to the bottom of the mouth, above the tongue; and
- turn the bottle upside down and slowly pour the compound, preferably comprised in a solution into the mouth so that the animal swallows it.

In another particular embodiment, the compound of the invention is present in a milk-based solution. For example, the compound may be mixed with milk and provided to piglets to treat and/or prevent a *C. difficile* infection. Alternatively, the compound may be comprised in a creep feed.

Compounds may be formulated into various pharmaceutical forms for administration purposes. As appropriate compositions there may be cited all compositions usually employed for systemically administering drugs. To prepare the pharmaceutical compositions of this invention, an effective amount of the particular compound, optionally in addition salt form or metal complex, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, particularly, for administration orally, rectally, percutaneously, or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, lubricants, binders, disintegrating agents and the like in the case of powders, pills, capsules, and tablets.

Because of their ease in administration, tablets and capsules represent a possible oral dosage unit forms, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations, which are intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin.

Compounds may also be administered via oral inhalation or insufflation by means of methods and formulations employed in the art for administration via this way. Compounds may be administered to the lungs in the form of a solution, a suspension or a dry powder, a solution being preferred. Any system developed for the delivery of solutions, suspensions or dry powders via oral inhalation or insufflation are suitable for the administration of compounds.

It is especially advantageous to formulate the aforementioned compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, suppositories, powder packets, wafers, injectable solutions or suspensions and the like, and segregated multiples thereof.

Therefore, the present invention also provides a pharmaceutical composition for use in the prevention and/or treatment of a C. *difficile* infection in a pig, preferably in a piglet, the composition comprising a compound as defined herein and a pharmaceutically acceptable carrier. In one embodiment, the pharmaceutical composition is in a unit dosage form. In a further embodiment, the unit dosage form comprises the daily dosage to be administered, in particular the daily dosages as defined herein before. More in particular, the pharmaceutical composition is in a unit dosage form comprising 10 to 1000 mg, preferably 50 to 500 mg, of the compounds as defined herein.

In a particular embodiment, the pharmaceutical composition is in a unit dosage form comprising 10 to 100 mg, preferably 25 to 75 mg, more preferably 40 to 60 mg, still more preferably 45 to 55 mg, still more preferably about 50 mg of the compound as defined herein.

In another particular embodiment, the pharmaceutical composition is in a unit dosage form comprising 100 to 1000 mg, preferably 250 to 750 mg, more preferably 400 to 600 mg, still more preferably 450 to 550 mg, still more preferably about 500 mg of the compound as defined herein.

In a particularly preferred embodiment of the present invention, the treatment and/or the prevention comprises orally administering the pharmaceutical composition comprising the compound as defined herein to piglets in a daily dose of 10 to 150 mg/kg bodyweight for at least 2 consecutive days.

All the preferred and particular embodiments of the use of the compound according to the present invention apply identically to the use of the pharmaceutical composition according to the present invention, i.e. comprising the compound as defined herein.

In a particular embodiment, the pharmaceutical composition is in a unit dosage form comprising 8 to 80 mg of moenomycins, preferably 20 to 60 mg, more preferably 30 to 50 mg, still more preferably about 40 mg.

In another particular embodiment, the pharmaceutical composition is in a unit dosage form comprising 80 to 800 mg of moenomycins, preferably 200 to 600 mg, more preferably 300 to 500 mg, still more preferably about 400 mg.

In a further embodiment, the pharmaceutical composition is in a unit dosage form comprising at least 4 mg of moenomycin A, preferably at least 10 mg, more preferably at least 15 mg, still more preferably about 20 mg.

In a particular embodiment, the pharmaceutical composition is in a unit dosage form comprising at least 40 mg of moenomycin A, preferably at least 100 mg, more preferably at least 150 mg, still more preferably about 200 mg.

In another embodiment, the pharmaceutical composition is in a unit dosage form comprising 8 to 80 mg of bambermycin, preferably 20 to 60 mg, more preferably 30 to 50 mg, still more preferably about 40 mg.

In another particular embodiment, the pharmaceutical composition is in a unit dosage form comprising 80 to 800 mg of bambermycin, preferably 200 to 600 mg, more preferably 300 to 500 mg, still more preferably about 400 mg.

As mentioned herein, the pharmaceutical composition is preferably an oral composition.

In a preferred embodiment of the present invention, the pharmaceutical composition is in the form of a solution.

More preferably, the pharmaceutical composition of the present invention is in the form of a solution and comprises between 5 and 25% w/v, preferably between 5 and 20% w/v, more preferably between 5 and 15% w/v, still more preferably about 10 % w/v of the compound as defined herein.

The present invention further relates to a composition for use in the treatment and/or prevention of a *C. difficile* infection in a pig, the composition comprising the compound as described above.

Preferably, the compound according to the invention is administered in combination with feed. Therefore, the present invention also provides a pig feed comprising a compounds as defined herein. The skilled person is well aware on how to convert daily dosage amounts to pig feed supplement concentrations and vice-versa.

All the preferred and particular embodiments of the use of the compound according to the present invention apply identically to the use of the pig feed composition according to the present invention, i.e. comprising the compound as defined herein.

The present invention further provides a method comprising mixing the compounds as described herein with pig feed.

The following examples are merely illustrative of some non-limiting embodiments of the present invention.

### EXAMPLES

### MATERIALS

*a) Objectives*
The objective of this nonpivotal study was to assess bambermycin in the treatment of or reduction in the incidence and severity of a CDI in newborn piglets.
*b) Investigational veterinary and control articles*
For all experiments described herein, the bambermycin used was the commercially available Flavomycin^{®}. Results were compared to a negative control group to estimate the appropriate dose. Bambermycin was provided in a 10% solution for the piglet model. The control for the piglet model was PBS.
*c) Study schedule*

**Table 1: Schedule of events**

| **Days post challenge (DPC)** | **Activity** | **General health observation** | **Clinical health observation** |
|---|---|---|---|
| - | Animal arrival and placement of sows into farrowing crates | X | |
| 0 | Farrowing | X | X |
| | Fecal samples TG01/TG02 piglets | | |
| | Challenge piglets with CD (TG01 and TG02) | | |
| | Treat piglets in TG02 - 4 hours post challenge | | |
| 1 | Treat piglets in TG02 - 24 hours post initial treatment | X | X |
| 2 | | X | X |
| 3 | | X | X |
| 4 | Fecal sample TG01 and TG02 piglets | X | X |
| | Necropsy piglets TG01 and TG02 (collect fixed intestine) | | |

*d) Study design*

### Experimental design:

This was a nonpivotal model development study involving the random assignment of piglets to two treatment groups: one group (TG01) was challenged with CD shortly after birth and then treated with a sham dose of PBS at 4 hours post challenge and again at 24 hours post initial treatment; one group (TG02) was challenged with CD shortly after birth and then treated with a soluble form of bambermycin at 4 hours post challenge and again at 24 hours post initial treatment.

All piglets were observed for clinical signs of CD infection from birth to DPC 4. Fecal samples were collected from all piglets DPC 0 (prior to challenge) and on all surviving piglets DPC 4 (end of study). The fecal samples were tested for CD toxin using an ELISA test. On DPC 4, all surviving piglets in TG01 and TG02 were euthanized and the intestines were observed for gross lesions. A formalin-fixed sample of intestine was collected for each piglet and submitted for histopathological examination.

### Treatment groups and number of animals:

The total number of bred sows needed for the study was three. Upon farrowing, the piglets were randomly assigned to TG01 or TG02 such that equal number of piglets were in each treatment group. Thirteen piglets per treatment group were enrolled in this study.

**Table 2: Treatment groups**

| **Treatment group** | **No. of sows/pigs** | **Treatment description** |
|---|---|---|
| TG01 | 3 sows: 13 piglets | Piglets challenged with CD shortly after birth and then treated with PBS at 4 hours post challenge and again at 24 hours post initial treatment |
| TG02 | 3 sows: 13 piglets | Piglets challenged with CD shortly after birth and then treated with a soluble form of bambermycin at 4 hours post challenge and again at 24 hours post initial treatment |

*Experimental units:*
   For treatment groups TG01 and TG02, the piglet was the experimental unit.
   *Randomization procedures:*
      Each newborn piglet was assigned a random number using the Excel random number generator function. Piglet identifications were listed on the allocation in numerical order and subsequently assigned to a treatment group such that 50% of each litter was assigned to each treatment.

### e) Study procedures

*Study animals:*

**Table 3: Description of study animals**

| **Category** | **Description** |
|---|---|
| Species | Porcine |
| Breed | Landrace |
| Number | ~3 near term first parity gilts and subsequent litters |
| Age | Various |
| Gender | Female - sows, male and female - piglets |
| Physiological status | Pregnant females |
| Health status | Healthy and free from apparent disease |

Litters from 3 first parity gilts were enrolled in the study.

Gilts were obtained from Wilson's Prairie View Swine, Burlington, WI. The Testing Site owned the animals. Animals were SPF and did not receive pre-farrowing vaccines for E. coli and other enteric diseases.

Each animal was identified by unique ear tag numbers.

The sows and litters were housed in individual farrowing crates.

All sows from the time of arrival to the day of farrowing were limit fed (~5 lbs per day) a commercially available nonmedicated swine gestation ration. Upon farrowing, each sow was fed a commercially available nonmedicated swine lactation ration. Sows were fed an amount each day at the judgement of the facility personnel. The amount fed was determined based on the body condition of the sow, number of piglets nursing, and on the consumption of the previous day's offering. Water was obtained from a source conventional to the test site and was offered *ad libitum* throughout the study.

Concurrently administered non-study treatments were not administered to study animals. Conditions of crates, feeders and waterers were checked daily. Daily high and low temperatures within the barn were recorded.

### Inclusion/exclusion criteria:

Piglets that were eligible for study enrollment met all the inclusion criteria outlined above. One piglet was excluded from the study after it was assessed by the Investigator and determined to be too weak for enrollment.

### Acclimation of test animals:

The pregnant gilts arrived on 29 January 2021. Farrowing dates are located in Table 4.

**Table 4: Parturition record.**

| **Sow ID** | **Farrow date** | **Number born** | | | **Litter live birth weight (Ib)** | |
|---|---|---|---|---|---|---|
| | | **Total** | **Alive** | **Dead** | **Total** | **Average** |
| 891 | 11 Feb. 2021 | 12 | 10 | 2 | 33.6 | 3.360 |
| 7824 | 12 Feb. 2021 | 5 | 4 | 1 | 11.7 | 2.925 |
| 7828 | 13 Feb. 2021 | 16 | 13 | 3 | 43.2 | 3.323 |

Animals did not receive any non-study medication or therapies during the acclimation or treatment phases of the study.

The pathologist examining the formalin fixed intestinal samples was masked to the treatment groups.

A qualified veterinarian conducted a routine postmortem examination (only if necessary) to determine cause of death for any animal that died spontaneously, or to characterize the circumstances requiring euthanasia prior to completion of the live phase of the study. Where possible, the examination occurred within 12 hours following death or euthanasia. Necropsy included a complete gross examination for pathologic changes.

### f) Description of study-specific procedures

### General health observations:

All sows and piglets were observed for general health observations daily. Sow #7824 had reduced appetite noted for the entire study. There were no additional general health observations noted.

### Body weight:

Piglets were weighed at birth and on DPC 4 (Tables 4 and 6).
*Clinical health and parturition observations:*
Upon parturition, the number of piglets born alive and dead were recorded on the Parturition Record. Daily clinical observations were conducted on piglets following the day of challenge. All animals were observed for the following parameters:

**Table 5: Daily clinical observations**

| | **Score** | | | |
|---|---|---|---|---|
| **Observation** | **0** | **1** | **2** | **3** |
| Body condition | Normal | Mildly thin | Gaunt | - |
| Behavior | Normal | Slightly depressed | Moderate depression | Severe depression |
| Feces | Normal | Mild diarrhea | Moderate diarrhea | Severe diarrhea |

*Fecal sampling and testing:*
   Fecal samples were collected from piglets on DPC 0 (prior to challenge) and DPC 4. The fecal samples were submitted to Iowa State University Veterinary Diagnostic Laboratory for testing the presence of CD toxins A and B using an ELISA test.
*Challenge:*
   On DPC 0, piglets assigned to groups TG01 and TG02 were inoculated intragastrically with 2mls of inoculum (Strain 645) containing 109/ml heat shocked CD spores.
*Treatment:*
   A soluble form of bambermycin was prepared by the sponsor. A 10% concentration was provided such that each piglet assigned to TG02 received approximately 500 mg of bambermycin orally at each dosing point. Due to limitations on the available amount of test article, three (3) piglets (#16, 18 and 19) received 50 mg of bambermycin at the second dosing for a total of 550 mg (not 1000 mg). Piglets assigned to TG01 received PBS.
*Removal of animals from study:*
   An enrolled piglet was only removed from further participation in the in-life phase of the study if it died spontaneously or was euthanized ahead of scheduled completion of the study. Animal removals are documented in Table 6. One piglet (#07; TG02) died after the initial dosing with bambermycin and it was determined upon necropsy that the piglet had aspirated the test article. This animal death was not considered challenge-related and was removed from the mortality data summarizations.
*Necropsy:*
   On DPC4, piglets were euthanized and necropsied. Any abnormal observations at necropsy were recorded, paying particular attention to abnormal gross lesions of the intestines. A section of intestine was collected and placed in formalin for histopathological examination by a pathologist.

### g) Animal disposition

Sows were euthanized at the conclusion of the study. The accountability and disposition of all enrolled animals were documented. Carcasses remaining after postmortem examination and sampling were disposed of according to local regulations. Animals were not rendered.

### RESULTS

The average body weight (lb) of each treatment group at farrowing and the average weight gain for the piglets that survived until the end of the assessment period are reported in Table 6.

**Table 6: Birth weight and weight gain for surviving piglets at 4 DPC.**

| **Treatment group** | **Average weight (Ib) at farrowing** | **Average weight gain (Ib) at DPC4** |
|---|---|---|
| TG01 | 3.2 | 0.03 |
| TG02 | 3.4 | 0.05 |

The challenge model was based on prior research in order to obtain a desired disease status. In this study, piglets began to show signs of a *C. difficile* infection 24 hours post-challenge as indicated by behavior, body condition and fecal scores. Mortality began within 24 hours of challenge as indicted in Tables 7 and 8.

**Table 7: Frequency table for survival days post-challenge.**

| **Treatment group** | **Survival days post-challenge** | | | |
|---|---|---|---|---|
| | **One** | **Two** | **Three** | **Four** |
| TG01 | 2 | 1 | 4 | 6 |
| TG02 | 2 | 1 | 1 | 8 |

Upon necropsy (both scheduled and unscheduled), the cecum and spiral colon were assessed and showed typical signs of a C. *difficile* infection (i.e., semi-liquid or pure liquid contents and mesocolonic edema; Table 8). Histopathological assessment indicated the presence of lesions in the colon (Table 8).

Fecal samples obtained prior to challenge and assessed for C. *difficile* toxin A/B were negative (Table 8). This test will detect toxin A at levels ≥ 0.8 ng/mL and toxin B at levels ≥ 2.5 ng/mL. The positive samples can be interpreted as a weak positive or strong positive depending on the color intensity 1₊, 2₊, 3₊, or 4₊, with 4₊ being the strongest. Eighty-three percent of the non-treated controls had the presence of C. *difficile* toxin A/B in their fecal samples compared to 0% in the bambermycin treatment group. In addition, 38% of the non-treated controls has lesions present in the colon vs 9% in the bambermycin treatment group.

In conclusion, the use of bambermycin in piglets is considered effective in reducing the incidence and severity of C. *difficile* infections in newborn piglets.

| **Pig ID** | **Treatment group** | **Date of death or euthanasia¹** | **Gross necropsy observations** | **Histopathology lesions (Y/N)²** | **DPC4 CD toxin A/B³** |
|---|---|---|---|---|---|
| 2 | TG01 | DPC1 | Pure liquid w/w/o blood | N | NA |
| 4 | TG01 | DPC4 | Semi liquid w/ mesocolonic edema | Y | 4₊ |
| 5 | TG01 | DPC4 | Semi liquid w/ mesocolonic edema | Y | 4₊ |
| 8 | TG01 | DPC3 | Semi liquid w/ mesocolonic edema | N (mild erosions) | NA |
| 9 | TG01 | DPC4 | Pure liquid w/w/o blood w/ mesocolonic edema | N (purulent peritonitis) | 3₊ |
| 12 | TG01 | DPC4 | Semi liquid | N | 0 |
| 14 | TG01 | DPC3 | Semi liquid | Y (autolysis) | NA |
| 15 | TG01 | DPC1 | Semi liquid | N | NA |
| 17 | TG01 | DPC4 | Semi liquid | Y | 4₊ |
| 20 | TG01 | DPC3 | Semi liquid | N | NA |
| 22 | TG01 | DPC3 | Semi liquid | N (autolysis) | NA |
| 23 | TG01 | DPC4 | Semi liquid w/ mesocolonic edema | Y | 4₊ |
| 25 | TG01 | DPC2 | Pure liquid w/w/o blood | N | NA |
| **Total** | | | | **5/13 (38%)** | **5/6 (83%)** |
| 1 | TG02 | DPC4 | Semi liquid | N | 0 |
| 3 | TG02 | DPC1 | Pure liquid w/w/o blood | N | NA |
| 6 | TG02 | DPC4 | Pure liquid w/w/o blood | N | 0 |
| 7 | TG02 | DPC1* | Normal (dosed in lung) | N (no lung lesions) | NA |
| 10 | TG02 | DPC4 | Pure liquid w/w/o blood | N | 0 |
| 11 | TG02 | DPC4 | Semi liquid | N | 0 |
| 13 | TG02 | DPC4 | Semi liquid | N | 0 |
| 16** | TG02 | DPC4 | Semi liquid | N | 0 |
| 18** | TG02 | DPC3 | Semi liquid | Y | NA |
| 19** | TG02 | DPC4 | Semi liquid | N | 0 |
| 21 | TG02 | DPC2 | Pure liquid w/w/o blood | - (autolysis) | NA |
| 24 | TG02 | DPC4 | Semi liquid | N | 0 |
| 26 | TG02 | DPC1 | Normal | N | NA |
| **Total** | | | | **1/11 (9%)** | **0/8 (0%)** |

| | | | | | |
|---|---|---|---|---|---|
| ¹ DPC = Days post-challenge (DPC4 is the end of the study). ² N = No lesions noted; Y = Multifocal to locally extensive, acute, necrotizing and purulent erosive and ulcerative colitis. ³ 0 = Negative; 3₊ = Strong positive; 4₊ = Strongest positive: NA = No sample taken due to premature death/euthanasia. * Not considered a challenge-related death. ** Received a total dose of 550 mg bambermycin. | | | | | |

### CONCLUSION

The objective of this nonpivotal study was to evaluate bambermycin in the treatment of or reduction in the incidence and severity of a CDI in newborn piglets.

In this study, piglets were randomly assigned to two treatment groups: one group (TG01) was challenged with CD shortly after birth and then treated with a sham dose of PBS at 4 hours post challenge and again at 24 hours post initial treatment; one group (TG02) was challenged with CD shortly after birth and then treated with a soluble form of bambermycin at 4 hours post challenge and again at 24 hours post initial treatment.

On DPC0, piglets assigned to groups TG01 and TG02 were inoculated intragastrically with 2mls of inoculum (Strain 645) containing 109/ml heat shocked CD spores.

A soluble form of bambermycin was prepared by the sponsor. A 10% concentration was provided such that each piglet assigned to TG02 received approximately 500 mg of bambermycin orally at each dosing point. Due to limitations on the available amount of test article, three (3) piglets (#16, 18 and 19) received 50 mg of bambermycin at the second dosing for a total of 550 mg (not 1000 mg). Piglets assigned to TG01 received PBS.

All piglets were observed for clinical signs of CD infection from birth to DPC4. Fecal samples were collected from all piglets DPC0 (prior to challenge) and on all surviving piglets DPC4 (end of study). The fecal samples were tested for CD toxin using an ELISA test. On DPC4, all surviving piglets in TG01 and TG02 were euthanized and the intestines were observed for gross lesions. A formalin-fixed sample of intestine was collected for each piglet and submitted for histopathological examination.

83% of the non-treated controls had the presence of C. *difficile* toxin A/B in their fecal samples compared to 0% in the bambermycin treatment group. In addition, 38% of the non-treated controls has lesions present in the colon vs 9% in the bambermycin treatment group.

Similar experiments wherein piglets were treated with two dosages of 500 mg or two dosages of 50 mg of bambermycin demonstrated far fewer lesions in both treatment groups in comparison to control. These experiments demonstrate that treatment with two dosages of 50 mg is effective.

Accordingly, the use of bambermycin in piglets significantly reduces the incidence and severity of C. *difficile* infections in newborn piglets.

## Claims

1. A compound for use in the treatment and/or prevention of a *Clostridium difficile* infection in a pig, wherein said compound is at least one of the compounds according to general formulae (I), (II), (III) or (IV), or the pharmaceutically acceptable salt, pharmaceutically acceptable solvate, isomer or mixture thereof: 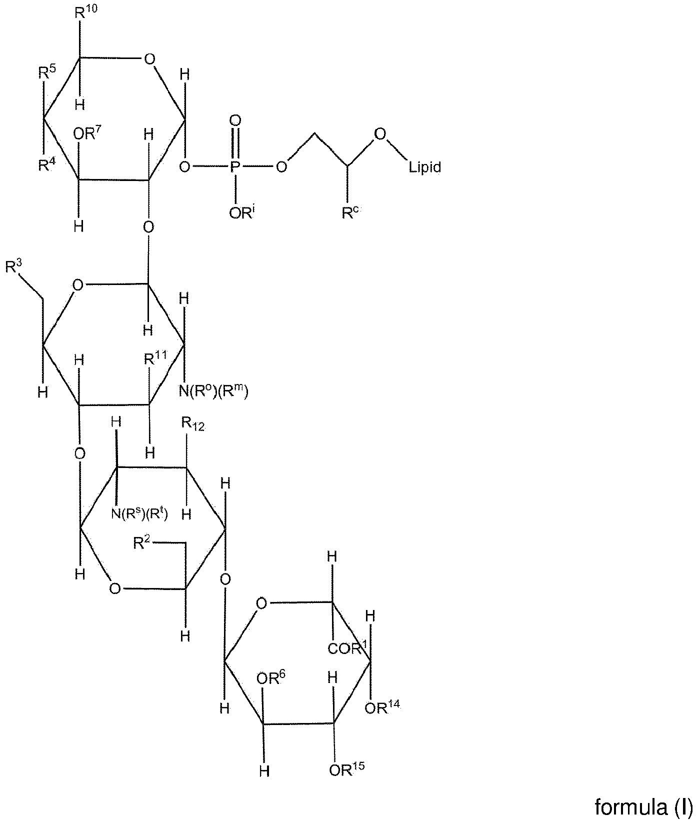 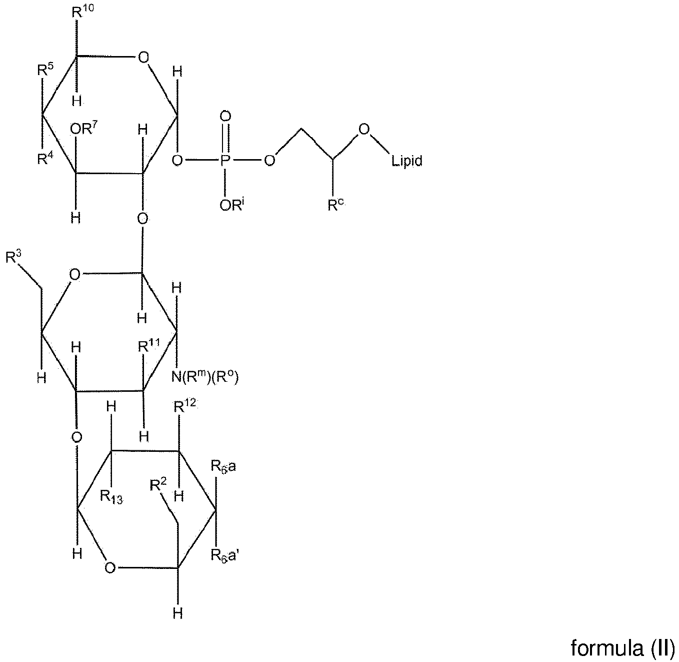 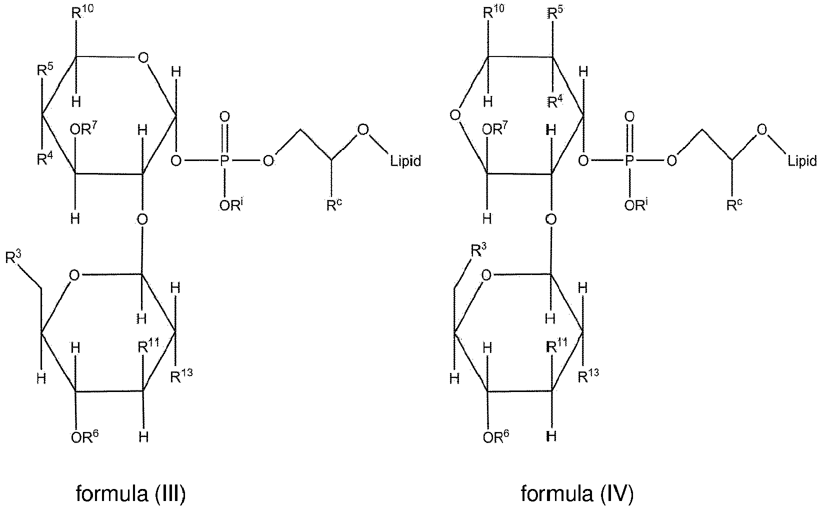 wherein
- each of R¹ is independently selected from -OH or -N(R^{1a})(R^{1b}), wherein each of R^{1a} and R^{1b}, independently from each other and at each occurrence, are selected from the group consisting of H, an amino protecting group, aryl, heteroaryl, aliphatic and a heteroaliphatic group,
- each of R², R⁴, R⁵, R¹¹ and R¹², independently from each other and at each occurrence, are selected from the group consisting of H, -OR^{z}, -N(R^{z})₂, aryl, heteroaryl, aliphatic, and a heteroaliphatic group, wherein R^{z} is independently selected from the group consisting of H, an hydroxyl protecting group, an amino protecting group, aryl, heteroaryl, aliphatic, heteroaliphatic and a carbohydrate moiety,
- each of R³ is independently selected from the group consisting of H, -OH, -NH₂, -SH, OR^{w}, - NH(R^{w}), -N(R^{w})₂, -SR^{w}, -O(C=O)R^{w}, -NH(C=O)R^{w}, -O(C=NH)R^{w}, -NH(C=NH)R^{w}, -S(C=NH))R^{w}, -NH(C=S)R^{w}, -S(C=O)R^{w}, -O(C=S)R^{w}, -S(=S)R^{w}, aryl, heteroaryl, aliphatic and a heteroaliphatic group, wherein R^{w} is selected from the group consisting of a carbohydrate moiety, aryl, heteroaryl, aliphatic, and heteroaliphatic group,
- each of R⁶, R¹⁴ and R¹⁵, independently from each other and at each occurrence are selected from the group consisting of H, hydroxyl protecting group, aryl, heteroaryl, aliphatic, heteroaliphatic group and carbohydrate moiety,
- each of R⁷ is independently selected from the group consisting of H, C(=O)N(R^{z'})₂, -C(=O)OR^{z'} , an hydroxyl protecting group, aryl, heteroaryl, aliphatic, heteroaliphatic group and carbohydrate moiety, wherein R^{z'} is independently selected from the group consisting of H, an hydroxyl protecting group, an amino protecting group, aryl, heteroaryl, aliphatic, and a heteroaliphatic group,
- each of R¹⁰ is independently selected from the group consisting of -C(=O)N(R^{l})(R^{p}), -C(=O)OR^{k}, and -CH₂OR^{k}, wherein R^{l} and R^{p} are independently from each other and at each occurrence selected from the group consisting of H, an amino protecting group, aryl, heteroaryl, aliphatic, heteroaliphatic group and a carbohydrate moiety, wherein R^{k} is independently selected from the group consisting of H, an hydroxyl protecting group, aryl, heteroaryl, aliphatic, heteroaliphatic group and a carbohydrate moiety,
- each of R¹³ is independently selected from the group consisting of -OH and -N(R^{o"})(R^{m"}) , wherein R^{o"} and R^{m"} independently from each other and at each occurrence, are selected from the group consisting of H, -C(=O)R^{h}, an amino protecting group, aryl, heteroaryl, aliphatic and heteroaliphatic group, wherein R^{h} is selected from the group consisting of a carbohydrate moiety, aryl, heteroaryl, aliphatic and heteroaliphatic group,
- each of (R°) and (R^{m}), independently from each other and at each occurrence, are selected from the group consisting of H, -C(=O)R^{w'}, an amino protecting group, aryl, heteroaryl, aliphatic and heteroaliphatic group, wherein R^{w'} is selected from the group consisting of a carbohydrate moiety, aryl, heteroaryl, aliphatic and heteroaliphatic group;
- each of (R^{s}) and (R^{t}), independently from each other and at each occurrence are selected from the group consisting of H, -C(=O)R^{w"}, an amino protecting group, aryl, heteroaryl, aliphatic and heteroaliphatic group, wherein R^{w"} is selected from the group consisting of a carbohydrate moiety, aryl, heteroaryl, aliphatic and heteroaliphatic group;
- each of R^{6a} and R^{6a'}, independently from each other and at each occurrence are selected from H or -OH, preferably R^{6a} is H and R^{6a'} is -OH;
- each of R^{c} is independently selected from the group consisting of H, halogen, heteroaryl, -OR^{q}, -N(R^{q})₂, -SR^{q}, NO₂, -NC, -CN, -N₃, -N(R^{q})=NR^{q}, -CHO, -C(=O)R^{q}, -C(=S)R^{q}, C(=NR^{q})R^{q}, - C(=O)OR^{q}, -C(=NR^{q})OR^{q}, -C(=NR^{q})N(R^{q})₂, -C(=O)N(R^{q})₂, -C(=S)OR^{q}, -C(=O)SR^{q}, -C(=S)SR^{q}, - P(=O)(OR^{q})₂, -S(=O)(OR^{q}), -S(=0)₂(OR^{q}), -P(=O)N(R^{q})₂, -P(=O)₂N(R^{q})₂, -C(=0)NR'S(=O)₂R^{q}, - S(=O)N(R^{q})₂ and -S(=O)₂N(R^{q})₂, wherein each of R^{q} is independently selected from the group consisting of H, aliphatic, heteroaliphatic, aryl, heteroaryl, and an hydroxyl protecting group,
- each of Rⁱ is independently selected from the group consisting of H, an hydroxyl protecting group, aliphatic, heteroaliphatic, aryl, and heteroaryl,
- each Lipid is independently selected from H or a C₁₋₃₀ aliphatic moiety, wherein 0 to 10 methylene units are optionally replaced with -O-, -NR^{x}-, -S-, -C(=O)-, -C(=NR^{x})-, -S(=O)-, - S(=O)₂-, -N=N-, -C=N-, -C(R^{y})=C(R^{y'})-, -N-O-, an arylene, or an heteroarylene moiety, wherein each of R^{x} is independently selected from the group consisting of H, aliphatic, heteroaliphatic, aryl, heteroaryl, or an amino protecting group, and wherein each of R^{y} and R^{y'}, independently from each other and at each occurrence are selected from the group consisting of H, aliphatic, heteroaliphatic, aryl and heteroaryl group.

2. The compound for use according to claim 1, wherein said compound is at least one of the compounds according to general formula (Ia), or the pharmaceutically acceptable salt, pharmaceutically acceptable solvate, isomer or mixture thereof, 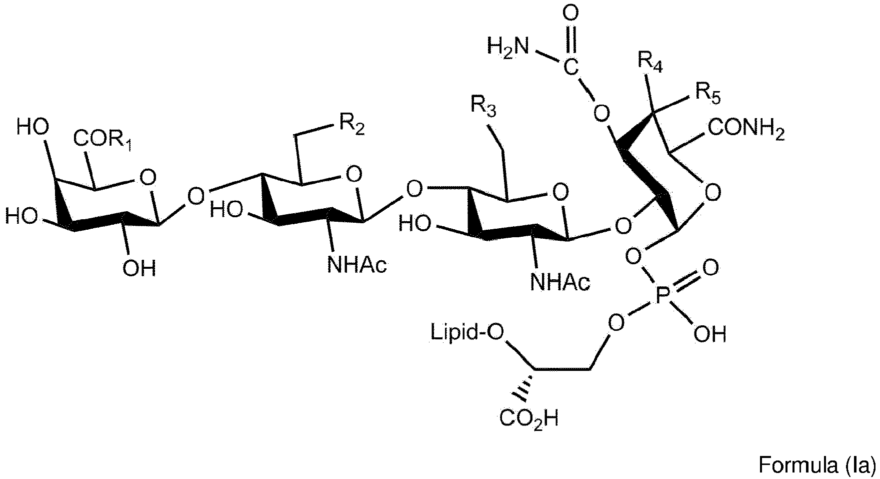 wherein
- each of R¹ is independently selected from OH, NH₂, or NH(R^{1b}), wherein R^{1b} is a C₁₋₁₀ cycloalkyl group or C₁₋₁₀ cycloalkenyl which is optionally substituted with an halide, hydroxyl or alkoxy, more preferably R¹ is -NH(R^{1b}), wherein R^{1b} is of following formula: 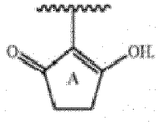
- each of R³ is independently selected from the group consisting of H, -OH and -OR^{w}, wherein R^{w} is D-glucose;
- each of R² and R⁴, independently from each other and at each occurrence are selected from H or -OH;
- each of R⁵ is independently selected from the group consisting of H, -OH and -CH₃;
- the Lipid is chosen among following Formula L₁ or Formula L₂:

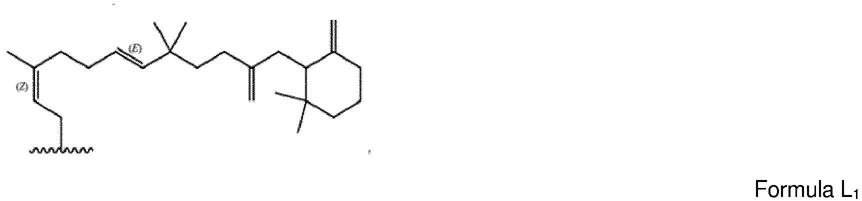

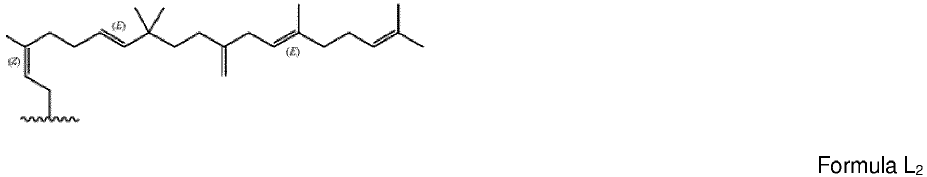

3. The compound for use according to claim 1 or claim 2, wherein said compound is at least one of the compounds according to general formula (Ib) or the pharmaceutically acceptable salt, pharmaceutically acceptable solvate, isomer or mixture thereof,

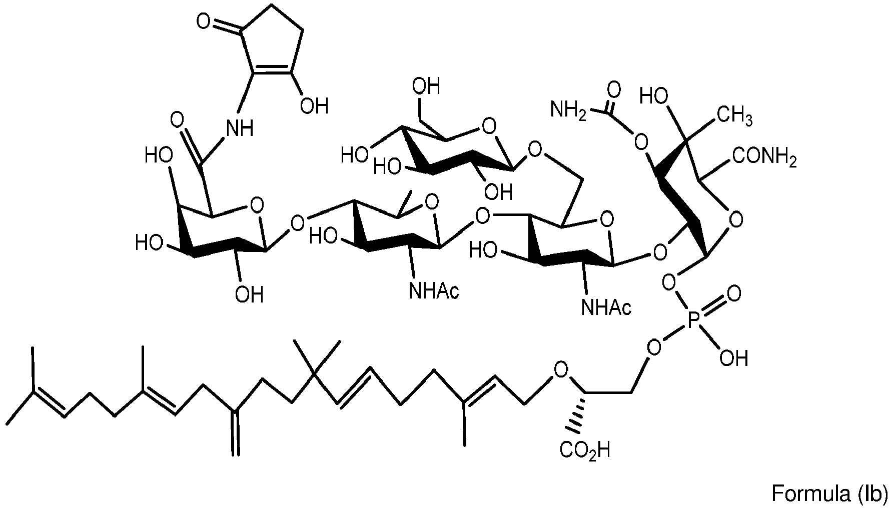

4. The compound for use according to claim 1, wherein said compound is at least one of the compounds according to formula (IIa) or formula (IIIa), or the pharmaceutically acceptable salt, pharmaceutically acceptable solvate, isomer or mixture thereof: 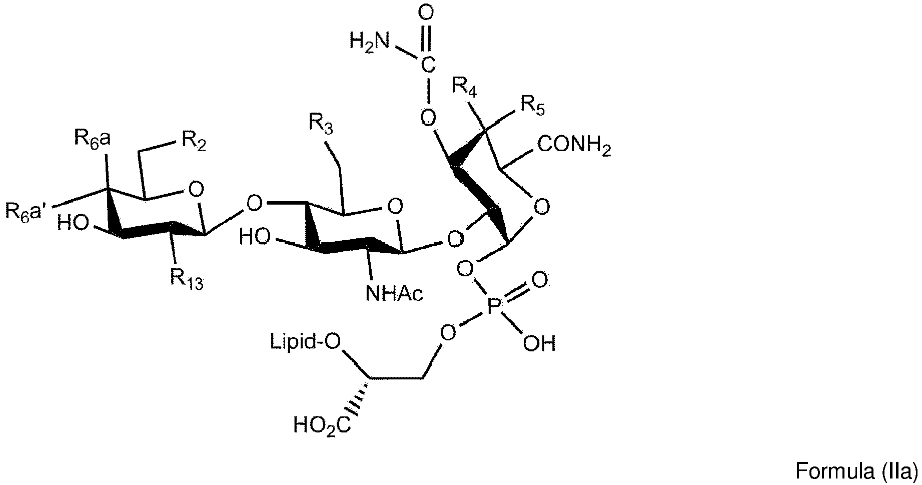 wherein
- R³ is H or -OH;
- each of R² and R⁴, independently from each other and at each occurrence, are selected from H or -OH;
- each of R^{6a} and R^{6a} independently from each other and at each occurrence, are selected from H or -OH, preferably R^{6a} is H and R^{6a'} is -OH;
- R⁵ is H, -OH or CH₃;
- R¹³ is -OH or -NHC(=O)CH₃
- the Lipid is chosen among following Formula L₃, Formula L₄ or Formula L₅: 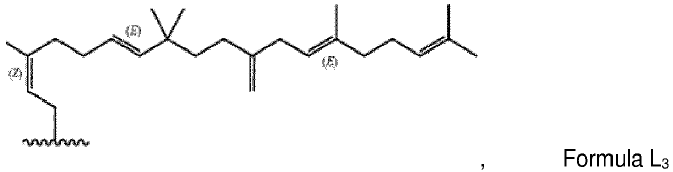 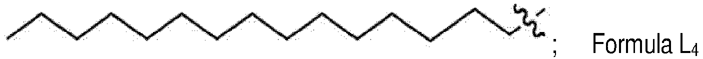 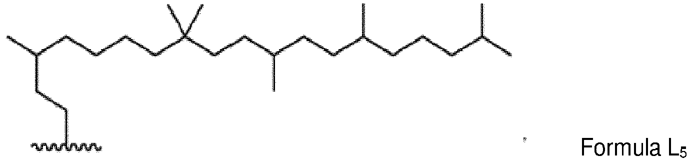 or 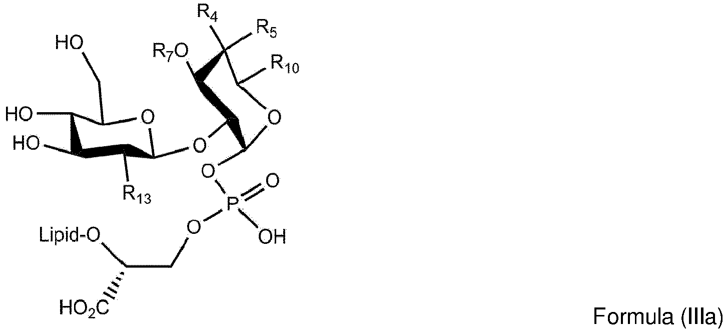
wherein
- each of R⁴ and R⁵, independently from each other and at each occurrence are selected from the group consisting of H, -CH₃ and -OH;
- R₇ is independently selected from H or -C(=O)NH₂, preferably, R₇ is C(=O)NH₂;
- R¹⁰ is independently selected from -C(=O)NH₂ or -C(=O)OH, preferably, R¹⁰ is C(=O)NH_{2.};
- R¹³ is independently selected from the group consisting of -OH or -NHC(=O)CH₃, preferably R¹³ is -NHC(=O)CH₃
- the Lipid is chosen among following Formula L₆, Formula L₇ or Formula L₈:

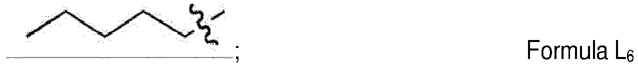

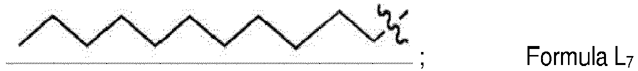

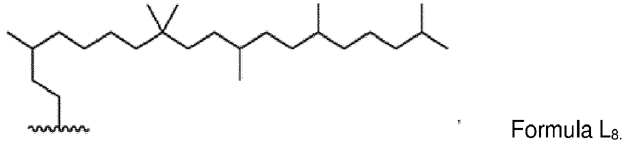

5. The compound for use according to any one of the previous claims, wherein the compound is administered to piglets, preferably to piglets being 15 days old or less.

6. The compound for use according to any one of the previous claims, wherein the treatment and/or prevention comprises administering a daily dose of 10 to 1000 mg, preferably 50 to 500 mg of the compound.

7. The compound for use according to any one of the previous claims, wherein the treatment and/or prevention comprises administering a daily dose of 2.5 to 350 mg/kg bodyweight, preferably of 10 to 150 mg/kg bodyweight.

8. The compound for use according to any one of the previous claims, wherein the compound is administered orally.

9. The compound for use according to any one of the previous claims, wherein the compound is administered at least 2 consecutive days.

10. A pharmaceutical composition for use in the treatment and/or prevention of a *Clostridium difficile* infection in a pig, the composition comprising a compound as defined in any one of claims 1 to 4 and one or more pharmaceutically acceptable carriers.

11. The pharmaceutical composition for use according to claim 10, wherein the composition comprises between 10 and 1000 mg, preferably between 50 and 500 mg of the compound as defined in any one of claim 1 to 4.

12. The pharmaceutical composition for use according to claim 10 or 11, wherein the treatment and/or the prevention comprises orally administering the compound as defined in any one of claims 1 to 4 to piglets in a daily dose of 10 to 1000 mg for at least 2 consecutive days.

13. A pig feed composition comprising a compound as defined in any one of claims 1 to 4 for use in the treatment and/or prevention of a *Clostridium difficile* infection in a pig.

14. The pharmaceutical or pig feed composition for use according to any one of claims 10 to 13, wherein the composition comprises bambermycin.
